(19) Europäisches Patentamt
European Patent Office
Office européen des brevets

(11) **EP 3 702 386 A1**

(12) **EUROPÄISCHE PATENTANMELDUNG**

(43) Veröffentlichungstag:
**02.09.2020  Patentblatt 2020/36**

(21) Anmeldenummer: **19159947.1**

(22) Anmeldetag: **28.02.2019**

(51) Int Cl.:
*C08G 18/08* (2006.01)     *A61F 13/472* (2006.01)
*A61F 13/537* (2006.01)    *A61F 13/539* (2006.01)
*A61L 15/22* (2006.01)     *C08G 18/28* (2006.01)
*C08G 18/40* (2006.01)     *C08G 18/44* (2006.01)
*C08G 18/48* (2006.01)     *C08G 18/72* (2006.01)
*C08G 18/73* (2006.01)     *C08G 18/75* (2006.01)
*C08G 18/79* (2006.01)     *A61F 13/00* (2006.01)
*A61F 13/515* (2006.01)    *C08G 101/00* (2006.01)
*A61F 13/15* (2006.01)     *A61F 13/511* (2006.01)
*A61F 13/514* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Covestro Deutschland AG**
**51373 Leverkusen (DE)**

(72) Erfinder:
• WEISER, Marc-Stephan
  **51515 Kürten-Dürscheid (DE)**
• PLUG, Sascha
  **51375 Leverkusen (DE)**
• DOERR, Sebastian
  **40593 Düsseldorf (DE)**

(74) Vertreter: **Levpat**
**c/o Covestro AG**
**Gebäude 4825**
**51365 Leverkusen (DE)**

(54) **VERBUNDSCHAUM FÜR HYGIENEARTIKEL**

(57)     Die Erfindung betrifft einen Hygieneartikel umfassend eine Absorptionsschicht und eine zumindest bereichsweise mit der Absorptionsschicht unmittelbar verbundene Speicherschicht, dadurch gekennzeichnet, dass die Absorptionsschicht einen Polyurethanschaum (A) umfasst, der durch mechanisches Aufschäumen einer wässrigen Polyurethandispersion a) und anschließendes Trocknen erhältlich ist und die Speicherschicht einen Polyurethanschaum (B) umfasst, wobei der Polyurethanschaum (B) durch reaktive Umsetzung wenigstens folgender Komponenten erhältlich ist:

A) Präpolymere erhältlich durch die Umsetzung von

A1) niedermolekularen Diisocyanaten einer Molmasse von 140 bis 278 g/mol, mit

A2) Polyalkylenoxiden mit einer OH-Funktionalität von zwei oder mehr,

A3) optional weitere isocyanatreaktive Komponenten, die nicht unter A2) fallen;

B) Wasser in einer Menge von mindestens 2 Gew.-%, bezogen auf das Gesamtgewicht der Zusammensetzung;

C) optional heterocyclische, 4-Ring- oder 6-Ring-Oligomere von niedermolekularen Diisocyanaten mit einer Molmasse von 140 bis 278 g/mol,

D) optional Katalysatoren;

E) optional Salze schwacher Säuren deren korrespondierende freie Säuren in Wasser bei 25 °C einen pKS-Wert von ≥ 3,0 und ≤ 14,0 aufweisen;

F) optional Tenside; und

G) optional ein- oder mehrwertige Alkohole oder Polyole;

H) optional hydrophile Polyisocyanate erhältlich durch Umsetzung von

H1) niedermolekularen Diisocyanaten einer Molmasse von 140 bis 278 g/mol und / oder daraus herstellbaren Polyisocyanaten mit einer Isocyanatfunktionalität von 2 bis 6, mit

H2) monofunktionellen Polyalkylenoxiden einer OH-Zahl von 10 bis 250, und einem Ethylenoxidanteil von 50 bis 100 mol%, bezogen auf die Gesamtmenge der enthaltenen Oxyalkylengruppen,

sowie ein Verfahren zur Herstellung des Hygieneartikels und dessen Verwendung.

EP 3 702 386 A1

**Beschreibung**

Die vorliegende Erfindung betrifft einen Hygieneartikel umfassend eine Absorptionsschicht und eine zumindest bereichsweise mit der Absorptionsschicht verbundene Speicherschicht. Weitere Gegenstände der Erfindung sind ein Verfahren zur Herstellung des erfindungsgemäßen Hygieneartikels.

In der europäischen Patentanmeldung EP 2 143 744 A1 sind hydrophile, aliphatische Polyurethanschäume beschrieben, die durch Umsetzung monomerarmer Prepolymere mit Wasser erhältlich sind. Das Absorptionsvermögen dieser Schäume für Flüssigkeiten wie Wasser ist zwar hoch, d.h. sie können große Mengen Flüssigkeit speichern, allerdings ist die Absorptionsgeschwindigkeit relativ niedrig, so dass nur vergleichsweise kleine Mengen Flüssigkeit pro Zeit aufgenommen werden können. Für Produkte mit dem Fokus einer schnellen Flüssigkeitsaufnahme wie z.B. Hygienebinden oder Windeln ist dies nachteilig, da es hier von entscheidender Bedeutung ist, dass die Flüssigkeit sehr schnell, quasi instantan, aufgenommen und von der Aufnahmestelle weg ins Innere des Schaums transportiert wird.

Aus der EP 2 028 223 A1 sind wiederum Polyurethanschäume bekannt, die durch mechanisches Aufschäumen von wässrigen Polyurethandispersionen hergestellt werden können. Diese Schäume sind unter anderem für die Verwendung in Wundauflagen vorgesehen und zeichnen sich durch eine hohe Absorptionsgeschwindigkeit für Flüssigkeiten aus. Allerdings ist ihr Absorptions- und Rückhaltevermögen für Flüssigkeiten begrenzt.

Die EP 2 140 888 A1 offenbart einen Verbundschaum, der insbesondere als Teil einer Wundauflage verwendet werden kann. Der Verbundschaum umfasst eine Speicherschicht, eine Absorptionsschicht und eine Deckschicht. Die Speicherschicht besteht aus einem Superabsorber und die Absorptionsschicht aus einem Polyurethanschaum, der durch mechanisches Aufschlagen einer wässrigen Polyurethandispersion erhalten wird. Bei dem Verbundschaum handelt es sich um einen Inselverbund, bei dem die Speicherschicht von der Absorptionsschicht und der Deckschicht vollständig umschlossen wird. Der Verbundschaum wird hergestellt, indem der Polyurethanschaum der Absorptionsschicht auf ein Substrat aufgetragen und ein Superabsorbervlies in den noch nassen Schaum gelegt wird. Nach der Trocknung des Polyurethanschaums wird dann die Deckschicht auf die Absorptionsschicht über dem Superabsorbervlies aufgebracht. Als Deckschicht können beispielsweise ein weiterer Polyurethanschaum oder eine thermoplastische Folie verwendet werden, die dann entweder durch Trocknung des Polyurethanschaums oder durch Thermolaminierung der thermoplastischen Folie mit der Absorptionsschicht verbunden werden. Verbundschäume für Wundauflagen ohne Deckschicht sind in der EP 2 140 888 A1 nicht offenbart. So ist die Deckschicht auch erforderlich da das Superabsorbervlies einerseits vor Zutritt von Flüssigkeit von außen geschützt werden muss und andererseits dessen direkter Kontakt mit Gewebe, etwa durch zufälliges Berühren, verhindert werden sollte. Aus diesem Grund sind die Gestaltungsmöglichkeiten bezüglich der räumlichen Anordnung und Verbindung der einzelnen Schichten bei dem bekannten Verbundschaum begrenzt.

Ein bisher nicht erreichtes Ziel bei der Herstellung von Schäumen, insbesondere bei deren Einsatz im hygienischen Bereich, ist es, verschiedene Eigenschaften, wie die Flexibilität des Schaums, die Absorptionsmenge und Absorptionsgeschwindigkeit (Wicking) von Flüssigkeiten, die Reißfestigkeit sowie ein nicht zu hohes oder zu niedriges Raumgewicht des Schaums gemeinsam zu optimieren und dabei die Quellung des entstehenden Schaumes zumindest in eine Raumrichtung der horizontalen Ebene zu minimieren. Nur so wäre es jedoch möglich einen möglichst reißfesten, aber dennoch flexiblen Schaum bereitzustellen, der dabei eine schnelle und hohe Absorption für Flüssigkeiten bei optimaler Raumdichte und möglichst geringer Ausdehnung aufweist. Insbesondere, um einen solchen Schaum beispielsweise für Hygieneartikel einsetzen zu können, wäre die Optimierung aller genannten Eigenschaften wünschenswert. Die Geschwindigkeit der Flüssigkeitsaufnahme ist dabei von besonderer Bedeutung. Bisher hat sich jedoch gezeigt, dass ein Schaum mit hoher Absorptionsfähigkeit für wässrige Medien (im Folgenden auch Flüssigkeitsabsorption genannt) eine eher niedrige Dichte, niedrige Flexibilität, eine niedrige Bruchspannung oder eine hohe Quellung aufweist. Gleichzeitig bewirken das hohe Aufnahmevermögen sowie die starke Quellung, dass sie Flüssigkeit nur langsam abgeführt wird und damit ein feuchtes Gefühl auf der Haut verbleibt. Dies ist für Anwendungen im Wound-Care Bereich gewünscht um für ein feuchtes Wundklima zu sorgen, im Bereich Hygieneanwendungen hingegen nicht. Daher besteht ein Bedarf, Schäume mit einer Kombination optimierter Eigenschaften bereitzustellen, insbesondere zur Anwendung in Hygieneartikeln. Mit Hygieneartikel sind hier vorwiegend Damenbinden, Windeln, Inkontinenzartikel und andere die Körperflüssigkeiten absorbierende Artikel gemeint.

Eine Aufgabe der Erfindung war es mindestens einen Nachteil des Standes der Technik mindestens zu einem Teil zu überwinden. Weiterhin war es Aufgabe der vorliegenden Erfindung einen Hygieneartikel bereit zu stellen, der ein hohes Absorptionsvermögen und eine hohe Absorptionsgeschwindigkeit aufweist und gleichzeitig eine große Flexibilität bei der Anordnung und Verbindung der einzelnen Schichten ermöglicht.

Zudem war es eine Aufgabe der vorliegenden Erfindung einen Hygieneartikel, eine Absorptionsschicht und eine Speicherschicht aufweisend, sowie ein geeignetes Verfahren zur Herstellung eines solchen Hygieneartikels bereitzustellen, wobei mindestens die Speicherschicht oder die Absorptionsschicht eine möglichst geringe Quellung in mindestens eine Raumrichtung bzw. eine möglichst geringe Volumenquellung bei weiterhin akzeptabler Absorptionsrate für Wasser, sowie eine ausreichende Flexibilität oder Bruchspannung aufweist. Die Absorptionsschicht, die Speicherschicht oder der Hygieneartikel sollte eine Quellung in mindestens eine Raumrichtung, bevorzugt in der horizontalen Ebene, von ≤ 40 %, bevorzugt von ≤ 38 %, oder bevorzugt von ≤ 35 %, oder bevorzugt von ≤ 30 % aufweisen. Insbesondere sollte der Schaum dabei eine Dichte von 60 g/L bis 300 g/L, oder bevorzugt im Bereich von 62 g/L bis 200 g/L aufweisen.

Weiterhin war es eine Aufgabe der Erfindung einen Zeit- und Kosten-sparenden Prozess bereitzustellen, um einen Hygieneartikel herzustellen.

Diese Aufgabe ist durch einen Hygieneartikel aufweisend einen Verbundschaum der eingangs genannten Art gelöst, wobei der Verbundschaum wenigstens eine Absorptionsschicht sowie eine Speicherschicht aus speziell ausgewählten Materialien aufweist. Insbesondere können bei der Herstellung des Hygieneartikels die Absorptionsschicht und die Speicherschicht auf eine Weise zusammengefügt werden, dass bei dem Herstellverfahren Zeit- und Kosten-sparend gearbeitet werden kann.

Ein erster Gegenstand der Erfindung ist ein Hygieneartikel umfassend eine Absorptionsschicht und eine zumindest bereichsweise mit der Absorptionsschicht unmittelbar verbundene Speicherschicht, dadurch gekennzeichnet, dass die Absorptionsschicht einen Polyurethanschaum (A) umfasst, der durch mechanisches Aufschäumen einer wässrigen Polyurethandispersion a) und anschließendes Trocknen erhältlich ist und die Speicherschicht einen Polyurethanschaum (B) umfasst, der durch reaktive Umsetzung wenigstens folgender Komponenten erhältlich ist:

A) Präpolymere erhältlich durch die Umsetzung von

A1) niedermolekularen Diisocyanaten einer Molmasse von 140 bis 278 g/mol, mit

A2) Polyalkylenoxiden mit einer OH-Funktionalität von zwei oder mehr,

A3) optional weitere isocyanatreaktive Komponenten, die nicht unter A2) fallen;

B) Wasser in einer Menge von mindestens 2 Gew.-%, bezogen auf das Gesamtgewicht der Zusammensetzung aus mindestens A) bis H);

C) optional heterocyclische, 4-Ring- oder 6-Ring-Oligomere von niedermolekularen Diisocyanaten mit einer Molmasse von 140 bis 278 g/mol,

D) optional Katalysatoren;

E) optional Salze schwacher Säuren deren korrespondierende freie Säuren in Wasser bei 25 °C einen pKS-Wert von ≥ 3,0 und ≤ 14,0 aufweisen;

F) optional Tenside; und

G) optional ein- oder mehrwertige Alkohole oder Polyole;

H) optional hydrophile Polyisocyanate erhältlich durch Umsetzung von

H1) niedermolekularen Diisocyanaten einer Molmasse von 140 bis 278 g/mol und / oder daraus herstellbaren Polyisocyanaten mit einer Isocyanatfunktionalität von 2 bis 6, mit

H2) monofunktionellen Polyalkylenoxiden einer OH-Zahl von 10 bis 250, und einem Ethylenoxidanteil von 50 bis 100 mol%, bezogen auf die Gesamtmenge der enthaltenen Oxyalkylengruppen.

So wurde überraschenderweise gefunden, dass der erfindungsgemäße Hygieneartikel einen Verbundschaum aus Absorptionsschicht und Speicherschicht aufweist, der sowohl dazu in der Lage ist Flüssigkeiten wie Wasser schnell aufzunehmen als auch in großen Mengen zu speichern. Der Hygieneartikel weist somit sowohl eine hohe Absorptionsgeschwindigkeit als auch ein hohes Absorptionsvermögen auf. Dieses Verhalten ist zumindest zum Teil dadurch begründet, dass die Absorptionsschicht und die Speicherschicht zumindest bereichsweise unmittelbar miteinander verbunden sind.

Unter "bereichsweise miteinander verbunden" wird im Rahmen der Erfindung verstanden, dass die Absorptionsschicht oder die Speicherschicht zu mindestens 20 %, bevorzugt zu mindestens 30 %, besonders bevorzugt zu mindestens 35 %, weiterhin bevorzugt zu mindestens 40 %, bezogen auf die Gesamtoberfläche mindestens einer der beiden Schichten, die sich bei der Herstellung ausbildet, mit der jeweils anderen Schicht verbunden ist. Bevorzugt ist die Absorptionsschicht als auch die Speicherschicht zu mindestens 20 %, bevorzugt zu mindestens 30 %, besonders bevorzugt zu mindestens 35 %, weiterhin bevorzugt zu mindestens 40 %, bezogen auf die jeweilige Gesamtoberfläche jeder der Schichten mit der Oberfläche der jeweils anderen Schicht verbunden. Unter der Gesamtoberfläche der Absorptionsschicht bzw. der Speicherschicht wird die gesamte Fläche verstanden, die sich bei der Herstellung des Hygieneartikels ausbildet, egal, ob diese Fläche mit einer anderen Schicht überdeckt ist oder frei liegt.

Unter "unmittelbar miteinander verbunden" wird im Rahmen der Erfindung verstanden, dass die Absorptionsschicht und die Speicherschicht ohne Aufbringen weiterer Schichten, wie beispielsweise einer zusätzlichen Klebeschicht, direkt miteinander verbunden sind.

Bevorzugt werden die Absorptionsschicht und die Speicherschicht auf einfache Weise in nahezu einem Schritt bzw. in direkt aufeinander folgenden Schritten unmittelbar aufeinander appliziert, sodass eine Zeit- und Kosten-effiziente Herstellung des erfindungsgemäßen Hygieneartikels möglich ist. Bevorzugt sind die Absorptionsschicht und die Speicherschicht eben ausgestaltet und mit einer ihrer ebenen Oberflächen miteinander verbunden.

In einer bevorzugten Ausführungsform des Hygieneartikels weisen die isocyanathaltigen Komponenten ausgewählt aus den Komponente A) bis H), insbesondere die Komponenten A), C) und H), insgesamt einen Isocyanatgehalt in einem Bereich von 2 bis 8 Gew.-% und einen Gehalt an Urethangruppen von 1,0 bis 3,5 mol/kg, jeweils bezogen auf die Gesamtmenge der isocyanathaltigen Komponenten, auf.

Bevorzugt weisen die isocyanathaltigen Komponenten zur Bildung des Polyurethanschaums (B), insbesondere die Komponenten A), C) und H), insgesamt einen Isocyanatgehalt in einem Bereich von 3 bis 7, oder bevorzugt in einem Bereich von 4 bis 6,5 Gew.-% und bevorzugt einen Gehalt an Urethangruppen in einem Bereich von 1,5 bis 3,0 mol/kg, oder bevorzugt in einem Bereich von 1,7 bis 2,8 mol/kg, jeweils bezogen auf die Gesamtmenge der isocyanathaltigen Komponenten, auf.

Bevorzugt weisen die eingesetzten Präpolymere A) einen Restmonomergehalt von unter 0,5 Gew.-%, bezogen auf die Gesamtmasse des Präpolymer A) auf. Dieser Gehalt kann durch entsprechend gewählte Einsatzmengen der Diisocyanate A1) und der Polyalkylenoxide A2) erreicht werden. Bevorzugt ist jedoch der Einsatz des Diisocyanats A1) im Überschuss und mit anschließender, bevorzugt destillativer, Abtrennung nicht umgesetzter Monomere.

Bei der Herstellung der isocyanatfunktionellen Präpolymere A) wird das Verhältnis der Polyalkylenoxide A2) zu den niedermolekularen, bevorzugt aliphatischen Diisocyanaten A1) typischerweise so eingestellt, dass auf 1 Mol OH-Gruppen des Polyalkylenoxide A2) 1,1 bis 20 Mol, bevorzugt mit 1,3 bis 5 Mol und besonders bevorzugt 1,5 bis 3,5 NCO-Gruppen des niedermolekularen, aliphatischen Diisocyanats A1) kommen.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140 °C, bevorzugt 60 bis 100 °C.

Bevorzugt werden vor, während und nach der Reaktion oder der destillativen Abtrennung des Diisocyanatüberschusses saure oder alkylierende Stabilisatoren, wie Benzoylchlorid, Isophthaloylchlorid, Methyltosylat, Chlorpropionsäure, HCl, Dibutylphosphat oder Antioxidantien wie Di-tert-butylkresol oder Tocopherol zugesetzt.

Der NCO-Gehalt der isocyanatfunktionellen Präpolymere A) beträgt bevorzugt 1,5 bis 8 Gew.-%, besonders bevorzugt 2 bis 7,5 Gew.-% und ganz besonders bevorzugt 3 bis 7 Gew.-%.

Das niedermolekulare Diisyocyanat A1) kann jedes Diisocyanat sein, dass der Fachmann hierfür einsetzen würde. Das Diisocyanat A1) kann entweder ein aliphatisches Diisocyanat sein oder ein aromatisches Diisocyanat oder ein Gemisch aus mindestens zwei Diisocyanaten aus diesen beiden Gruppen.

Beispiele für niedermolekulare, aliphatische Diisocyanate der Komponente A1) sind Hexamethylendiisocyanat (HDI), Isophorondiisocyanat (IPDI), Butylendiisocyanat (BDI), Pentamethylendiisocyanat (PDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI), Pentamethylendiisocyanat (PDI), und Bis(isocyanatocyclohexyl)methan (HMDI) bevorzugt sind. Besonders bevorzugt sind PDI, HDI, IPDI, ganz besonders bevorzugt Hexamethylendiisocyanat und Pentamethylendiisocyanat oder eine Mischung aus mindestens zwei hiervon.

Werden aromatische Diisocyanate als Komponente A1) eingesetzt so handelt es sich bevorzugt um Toluol-2,4-diisocyanat (2,4-TDI), Toluol-2,6-diisocyanat (2,6-TDI) oder 2,2'-Methylendiphenyldiisocyanat (2,2'-MDI), 2,4-Methylendiphenyldiisocyanat (2,4'-MDI), 4,4'-Methylendiphenyldiisocyanat (4,4'-MDI), oder Mischungen aus mindestens zwei hiervon.

Werden aromatische Diisocyanate als Komponente A1) eingesetzt so handelt es sich bevorzugt um Toluol-2,4-diisocyanat (2,4-TDI), Toluol-2,6-diisocyanat (2,6-TDI) oder 2,2'-Methylendiphenyldiisocyanat (2,2'-MDI), 2,4'-Methylendiphenyldiisocyanat (2,4'-MDI), 4,4'-Methylendiphenyldiisocyanat (4,4'-MDI) oder Mischungen aus mindestens zwei hiervon.

Polyalkylenoxide der Komponente A2) können alle Polyalkylenoxide sein, die der Fachmann hierfür verwenden würde. Beispiele hierfür sind ausgewählt aus der Gruppe bestehend aus Polyethylenoxid, Polypropylenoxid, Polytetrahydrofuran oder einer Mischung aus mindestens zwei hiervon. Polyalkylenoxide der Komponente A2) sind bevorzugt Copolymere aus Ethylenoxid und Propylenoxid mit einem Ethylenoxidgehalt, bezogen auf die Gesamtmenge der enthaltenen Oxyalkylengruppen, von 50 bis 100 mol%, bevorzugt von 60 bis 90 mol%, gestartet auf Polyolen oder Aminen. Bevorzugte Starter dieser Art sind ausgewählt aus der Gruppe bestehend aus Ethylenglycol, 1,3-Propandiol, 1,4-Butandiol, Glycerin, Trimethylolpropan (TMP), Sorbit, Pentaerythrit, Triethanolamin, Ammoniak und Ethylendiamin oder eine Mischung aus mindestens zwei hiervon.

Die Polyalkylenoxide der Komponente A2) besitzen typischerweise zahlenmittlere Molekulargewichte von 250 bis 10000 g/mol, bevorzugt von 300 bis 2800 g/mol, oder bevorzugt 350 bis 1500 g/mol. Ferner besitzen die Polyalkylenoxide der Komponente A2) OH-Funktionalitäten von 2 bis 6, bevorzugt von 2 bis 5, besonders bevorzugt von 2 bis 4.

Bevorzugt werden für die Komponente A3) grundsätzlich alle dem Fachmann an sich bekannten ein- und mehrwertigen Alkohole oder Amine sowie Mischungen hieraus eingesetzt. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole oder Mischungen von mindestens zwei hiervon. Beispiele für ein- oder mehrwertige Amine seien Butylamin Ethylendiamin oder aminterminierte Polyalkylenglycole (z.B. Jeffamine®) genannt.

Bei der Herstellung des Präpolymer A) können Katalysatoren eingesetzt, wie sie beispielsweise für Komponente D) beschrieben werden.

Das als Komponente B) einzusetzende Wasser kann als solches, als Kristallwasser eines Salzes, als Lösung in einem dipolar-aprotischen Lösungsmittel oder auch als Emulsion eingesetzt werden. Bevorzugt wird das Wasser als solches oder in einem dipolar-aprotischen Lösungsmittel eingesetzt. Ganz besonders bevorzugt wird das Wasser als solches eingesetzt.

Gegebenenfalls einzusetzende Verbindungen der Komponente C) sind heterocyclische, 4- Ring- oder 6-Ring-Oligomere von niedermolekularen Diisocyanaten mit einer Molmasse von 140 bis 278 g/mol wie Isocyanurate, Iminooxadiazindione oder Uretdione der vorgenannten niedermolekularen Diisocyanate. Bevorzugt werden für Komponente C) aliphatische Diisocyanate verwendet. Bevorzugt sind heterocyclische 4-Ring-Oligomere wie Uretdione. Bevorzugt weisen die 4-Ring- oder 6-Ring-Oligomere von niedermolekularen Diisocyanaten eine Funktionalität in einem Bereich von 2 bis 6, oder bevorzugt in einem Bereich von 2,1 bis 5,5, oder bevorzugt in einem Bereich von 2,5 bis 5 auf.

Beispiele für niedermolekulare, aliphatische Diisocyanate der Komponente C) sind Hexamethylendiisocyanat (HDI), Isophorondiisocyanat (IPDI), Butylendiisocyanat (BDI), Pentamethylendiisocyanat (PDI), Bisisocyanatocyclo-hexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI), Pentamethylendiisocyanat (PDI), und Bis(isocyanatocyclohexyl)methan (HMDI) bevorzugt sind. Besonders bevorzugt sind PDI, HDI, IPDI, ganz besonders bevorzugt Hexamethylendiisocyanat und Pentamethylendiisocyanat.

Werden aromatische Diisocyanate als Komponente C) eingesetzt so handelt es sich bevorzugt um Toluol-2,4-diisocyanat (2,4-TDI), Toluol-2,6-diisocyanat (2,6-TDI) oder 2,2'-Methylendiphenyldiisocyanat (2,2'-MDI), 2,4'-Methylendiphenyldiisocyanat (2,4'-MDI), 4,4'-Methylendiphenyldiisocyanat (4,4'-MDI), oder Mischungen aus mindestens zwei hiervon.

Der durch den Einsatz der Komponente C) erhöhte Gehalt an Isocyanatgruppen sorgt für ein besseres Aufschäumen, da bei der Isocyanat-Wasser-Reaktion mehr $CO_2$ gebildet wird.

Zur Beschleunigung der Urethanbildung können in Komponente D) Katalysatoren eingesetzt werden. Dabei handelt es sich typischerweise um die dem Fachmann aus der Polyurethantechnologie bekannten Verbindungen. Bevorzugt sind hier Verbindungen der Gruppe bestehend aus katalytisch aktiven Metallsalzen, Aminen, Amidinen und Guanidinen. Beispielhaft zu nennen sind Zinndibutyldilaurat (DBTL), Zinnacetat, 1,8-Diazabicyclo[5.4.0]undecen-7 (DBU), 1,5-Diazabicyclo[4.3.0]nonen-5 (DBN), 1,4-Diazabicyclo[3.3.0]octen-4 (DBO), N-Ethylmorpholin (NEM), Triethylendiamin (DABCO), Pentamethylguanidin (PMG), Tetrametylguanidin (TMG), Cyclotetramethylguanidin (TMGC), n-Decyltetramethylguanidin (TMGD), n-Dodecyltetramethylguanidin (TMGDO), Dimethylaminoethyltetramethylguanidin (TMGN), 1,1,4,4,5,5-Hexamethylisobiguanidin (HMIB), Phenyltetramethylguanidin (TMGP) und Hexamethylenoctamethylbiguanidin (HOBG).

Bevorzugt ist der Einsatz von Aminen, Amidinen, Guanidinen oder deren Mischungen als Katalysatoren der Komponente D). Weiterhin bevorzugt ist auch die Verwendung von 1,8-Diazabicyclo[5.4.0]undecen-7 (DBU).

Bevorzugt wird bei der Umsetzung der Komponenten zur Herstellung der Polyurethanschäume (A) und B), insbesondere zur Herstellung des Polyurethanschaums (B) ganz auf Katalysatoren verzichtet.

Gegebenenfalls können als Komponente E) Salze schwacher Säuren verwendet werden, deren korrespondierende freie Säuren in Wasser bei 25 °C einen pKS-Wert von ≥ 3,0 und ≤ 14,0 aufweisen. Beispiele geeigneter Salze schwacher Säuren sind Kaliumhydroxid, Natriumhydroxid, Kaliumcarbonat, Natriumcarbonat und Natriumhydrogencarbonat, Natriaumacetat, Kaliumacetat, Natriumcitrat, Kaliumcitrat, Natriumbenzoat, Kaliumbenzoat, wobei auch beliebige Mischungen dieser Salze mit umfasst sind. Bevorzugt ist, wenn die Salze schwacher Säuren aus der Gruppe Natriumhydroxid, Natriumhydrogencarbonat und Natriumcarbonat ausgewählt sind. In diesem Fall resultiert eine besonders kurze Reaktionszeit.

Zur Verbesserung der Schaumbildung, Schaumstabilität oder der Eigenschaften des resultierenden Polyurethanschaums, können Verbindungen der Komponente F) eingesetzt werden, wobei solche Additive grundsätzlich alle an sich bekannten anionischen, kationischen, amphoteren und nichtionischen Tenside sowie Mischungen hieraus sein können. Bevorzugt werden Alkylpolyglycoside, EO/PO-Blockcopolymere, Alkyl- oder Arylalkoxylate, Siloxanalkoxylate, Ester der Sulfobernsteinsäure und/oder Alkali- oder Erdalkalimetallalkanoate oder Mischungen von mindestens zwei hiervon eingesetzt. Besonders bevorzugt werden EO/PO-Blockcopolymere eingesetzt. Bevorzugt werden allein die EO/PO-Blockcopolymere als Komponente F) eingesetzt.

Zudem können zur Verbesserung der Schaumeigenschaften des resultierenden Polyurethanschaums Verbindungen der Komponente G) verwendet werden. Hierbei handelt es sich um grundsätzlich alle dem Fachmann an sich bekannten ein- und mehrwertigen Alkohole sowie Mischungen hieraus. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole oder Mischungen von mindestens zwei hiervon.

Bei der Herstellung der hydrophilen Polyisocyanate H) wird das Verhältnis der monofunktionellen Polyalkylenoxide H2) zu den niedermolekularen Diisocyanaten H1) typischerweise so eingestellt, dass auf 1 Mol OH-Gruppen der monofunktionellen Polyalkylenoxide 1,25 bis 20 Mol, bevorzugt mit 2 bis 15 Mol und besonders bevorzugt 5 bis 13 Mol NCO-Gruppen des niedermolekularen Diisocyanats H1) kommen. Anschließend erfolgt die Allophanatisierung bzw. Biurethisierung und/oder Isocyanuratbildung bzw. Uretdionbildung. Werden die Polyalkylenoxide H2) über Urethangruppen an die bevorzugt aliphatischen Diisocyanate H1) gebunden, findet bevorzugt im Anschluss eine Allophanatisierung statt. Weiterhin ist bevorzugt, dass Isocyanurat-Struktureinheiten gebildet werden.

Eine bevorzugte alternative Herstellung der hydrophilen Polyisocyanate H) erfolgt typischerweise durch Umsetzung von 1 Mol OH-Gruppen der monofunktionellen Polyalkylenoxidkomponente H2) mit 1,25 bis 20 Mol, bevorzugt mit 2 bis 15 Mol und besonders bevorzugt 5 bis 13 NCO-Gruppen eines Polyisocyanates H1) mit einer Isocyanatfunktionalität von 2 bis 6, basierend auf aliphatischen oder aromatischen Diisocyanaten, bevorzugt aliphatischen Diisocyanaten. Beispielhaft für derartige Polyisocyanate H1) sind Biuret-Strukturen, Isocyanurate bzw. Uretdione basierend auf bevorzugt aliphatischen Diisocyanaten. Dabei werden das Polyisocyanat H1) und das Polyalkylenoxid H2) bevorzugt über eine Urethangruppe bzw. eine Harnstoffgruppe miteinander verknüpft, wobei besonders die Verknüpfung über Urethangruppen bevorzugt ist.

Bevorzugt werden ausschließlich aliphatische Diisocyanate bei der Herstellung erfindungsgemäßer Hygieneartikel, insbesondere für die Herstellung der Absorptionsschicht und der Speicherschicht eingesetzt. Insbesondere werden bevorzugt ausschließlich aliphatische Diisocyanate für die Komponenten A1), C) und H1) eingesetzt.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140 °C, bevorzugt bei 60 bis 100 °C.

Wurde mit überschüssigem niedermolekularen Diisocyanat gearbeitet, erfolgt anschließend die Entfernung des Überschusses an niedermolekularem, aliphatischen Diisocyanat bevorzugt durch Dünnschichtdestillation.

Vor, während und/oder nach der Reaktion oder der destillativen Abtrennung des Diisocyanatüberschusses können saure oder alkylierende Stabilisatoren, wie Benzoylchlorid, Isophthaloylchlorid, Methyltosylat, Chlorpropionsäure, HCl oder Antioxidantien, wie Di-*tert*-butylkresol oder Tocopherol zugesetzt werden.

Der NCO-Gehalt der hydrophilen Polyisocyanate H) beträgt bevorzugt 0,3 bis 23 Gew.-%, besonders bevorzugt 2 bis 21 Gew.-% und ganz besonders bevorzugt 3 bis 18 Gew.-%.

Beispiele für niedermolekulare, aliphatische Diisocyanate der Komponente H1) sind Hexamethylendiisocyanat (HDI), Isophorondiisocyanat (IPDI), Butylendiisocyanat (BDI), Pentamethylendiisocyanat (PDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI), Pentamethylendiisocyanat (PDI), und Bis(isocyanatocyclohexyl)methan (HMDI) bevorzugt sind. Besonders bevorzugt sind PDI, HDI, IPDI, ganz besonders bevorzugt Hexamethylendiisocyanat und Pentamethylendiisocyanat.

Werden aromatische Diisocyanate als Komponente H1) eingesetzt so handelt es sich bevorzugt um Toluol-2,4-diisocyanat (2,4-TDI), Toluol-2,6-diisocyanat (2,6-TDI) oder 2,2'-Methylendiphenyldiisocyanat (2,2'-MDI), 2,4'-Methylendiphenyldiisocyanat (2,4'-MDI), 4,4'-Methylendiphenyldiisocyanat (4,4'-MDI), oder Mischungen aus mindestens zwei hiervon.

Beispiele für höhermolekulare Polyisocyanate H2) sind Polyisocyanate mit einer Isocyanatfunktionalität von 2 bis 6 mit Isocyanurat-, Urethan-, Allophanat-, Biuret-, Iminooxadiazintrion-, Oxadiazintrion- und/oder Uretdiongruppen auf Basis der im vorstehenden Abschnitt genannten aliphatischen und/oder cycloaliphatischen Diisocyanate.

Bevorzugt werden als Komponente H2) höhermolekulare Verbindungen mit Biuret-, Iminooxadiazindion-, Isocyanurat- und/oder Uretdiongruppen auf Basis von Hexamethylendiisocyanat, Isophorondiisocyanat und/oder 4,4'-Diisocyanatodicyclohexylmethan eingesetzt. Weiter bevorzugt sind Isocyanurate. Ganz besonders bevorzugt sind Strukturen auf Basis von Hexamethylendiisocyanat.

Die monofunktionellen Polyalkylenoxiden H2) weisen eine OH-Zahl von 15 bis 250, bevorzugt von 28 bis 112, und einem Ethylenoxidanteil von 50 bis 100 mol%, bevorzugt von 60 bis 100 mol%, bezogen auf die Gesamtmenge der enthaltenen Oxyalkylengruppen auf.

Unter monofunktionellen Polyalkylenoxid im Sinne der Erfindung sind Verbindungen zu verstehen, die nur eine isocyanatreaktive Gruppe, d.h. eine Gruppe, die mit einer NCO-Gruppe reagieren kann, aufweisen.

Die Herstellung von Polyalkylenoxiden H2) durch Alkoxylierung geeigneter Startermoleküle ist literaturbekannt (z.B. Ullmanns Encyclopädie der technischen Chemie, 4. Auflage, Band 19, Verlag Chemie, Weinheim S. 31-38). Geeignete Startermoleküle sind insbesondere gesättigte Monoalkohole wie Methanol, Ethanol, n-Propanol, Isopropanol, n-Butanol, Isobutanol, sec-Butanol, Diethylenglykolmonobutylether sowie aromatische Alkohole wie Phenol oder Monoamine wie Diethylamin. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Die monofunktionellen Polyalkylenoxide H2) besitzen typischerweise zahlenmittlere Molekulargewichte von 220 bis 3700 g/mol, bevorzugt von 250 bis 2800 g/mol, oder bevorzugt von 300 bis 2000 g/mol.

Die monofunktionellen Polyalkylenoxide H2) besitzen bevorzugt eine OH-Gruppe als isocyanatreaktive Gruppe.

Typischerweise werden die Komponenten A) bis H) in folgenden Mengen eingesetzt:

100 Gewichtsteile isocyanatfunktioneller Präpolymere A)

0,1 bis 200 Gewichtsteile Wasser B)

0 bis 30 Gewichtsteile heterocyclischer Oligomere C)

0 bis 1 Gewichtsteile an Katalysatoren D)

0 bis 5 Gewichtsteile an Salzen schwacher Säuren deren korrespondierende freie Säuren in Wasser bei 25 °C einen pKS-Wert von ≥ 3,0 und ≤ 14,0 aufweisen E)

0 bis 10 Gewichtsteile Tenside F)

0 bis 20 Gewichtsteile Alkohole G)

0,5 bis 50 Gewichtsteilen hydrophile Polyisocyanatkomponente H)

Bevorzugt werden die Komponenten A) bis H) in folgenden Mengen eingesetzt:

100 Gewichtsteile isocyanatfunktioneller Präpolymere A)

0,1 bis 100 Gewichtsteile Wasser B)

1 bis 20 Gewichtsteile heterocyclischer Oligomere C)

0 bis 1 Gewichtsteile an Katalysatoren D)

0 bis 5 Gewichtsteile an Salzen schwacher Säuren deren korrespondierende freie Säuren in Wasser bei 25 °C einen pKS-Wert von ≥ 3,0 und ≤ 14,0 aufweisen E)

0 bis 5 Gewichtsteile Tenside F)

0 bis 10 Gewichtsteile Alkohole G)

1 bis 25 Gewichtsteilen hydrophile Polyisocyanate H)

Besonders bevorzugt werden die Komponenten A) bis H) in folgenden Mengen eingesetzt:

100 Gewichtsteile isocyanatfunktioneller Präpolymere A)

1 bis 60 Gewichtsteile Wasser B)

2 bis 15 Gewichtsteile heterocyclischer Oligomere C)

0 bis 0,5 Gewichtsteile an Katalysatoren D)

0 Gewichtsteile an Salzen schwacher Säuren deren korrespondierende freie Säuren in Wasser bei 25 °C einen pKS-Wert von $\geq$ 3,0 und $\leq$ 14,0 aufweisen E)

0 bis 3 Gewichtsteile Tenside F)

0 Gewichtsteile Alkohole G)

2 bis 15 Gewichtsteilen hydrophile Polyisocyanate H).

Bevorzugt weist die Komponente A) einen Gewichtsanteil an niedermolekularen Diisocyanaten mit einer Molmasse von 140 bis 278 g/mol von unter 1,0 Gew.-%, bevorzugt von unter 0,5 Gew.-%, bevorzugt von unter 0,3 Gew.-%, oder bevorzugt von unter 0,1 Gew.-%, bezogen auf das Präpolymer auf. Die Einstellung des Gewichtsanteils an niedermolekularen Diisocyanaten erfolgt bevorzugt über Destillation.

Bevorzugt werden aliphatische Isocyanate als Komponente A1) eingesetzt.

Bevorzugt werden zur Herstellung des Polyurethanschaums (B) mindestens folgende Schritte durchgeführt:

I) Herstellen des Präpolymers A) aus den Komponenten A1), A2) und gegebenenfalls A3), D),

II) Optionales Mischen der Komponenten A), C) und H) sowie anderer isocyanathaltiger Komponenten unter Erhalt einer Präpolymer-Mischung,

III) Optional Zugabe von A3) und gegebenenfalls D),

IV) Optionales Mischen der Komponente B) mit allen weiteren Komponenten, insbesondere D), E), F) und G) außer der Präpolymer-Mischung,

V) Mischen der unter I) bis III) erhaltenen Präpolymer-Mischung mit der Mischung aus IV).

Bevorzugt wird die Temperatur bei mindestens einem der Schritte I) bis V) in einem Bereich von 2 bis 70°C, oder bevorzugt in einem Bereich von 10 bis 50 °C, oder bevorzugt von 23 (RT) bis 40 °C gewählt, wobei RT für Raumtemperatur steht.

Die Mischung kann im Anschluss an Schritt V) auf ein Substrat aufgegeben und aushärten gelassen werden. Das Aushärten wird bevorzugt bei einer Temperatur in einem Bereich von 20 bis 50 °C durchgeführt. Unter zu Hilfenahme von Konvektionsöfen oder Infrarottrocknern kann das Aushärten und eine gleichzeitige Trocknung auch bei höheren Temperaturbereichen, z.B. zwischen 50 und 200 °C durchgeführt werden. Ebenso ist es möglich, das Aushärten bei einer geringeren Temperatur und das Trocknen in einem zweiten Schritt oder weiteren Verlauf bei höherer Temperatur durchzuführen.

Die Herstellung der Polyurethanschäume (B) erfolgt bevorzugt durch Mischen der Komponenten A), hergestellt aus den Komponenten A1), A2) und gegebenenfalls A3), D), gegebenenfalls mit C) und/oder H) in beliebiger Reihenfolge, sowie anschließend mit einer Mischung aus B) und gegebenenfalls D), E), F), G), Aufschäumen der Mischung und Aushärtung, bevorzugt durch chemische Vernetzung. Bevorzugt werden die Komponenten A), C) und H) miteinander vorvermischt. Die gegebenenfalls einzusetzenden Salze E) und gegebenenfalls die Tenside F) werden bevorzugt in Form ihrer wässrigen Lösungen dem Reaktionsgemisch zugesetzt.

Das Aufschäumen kann dabei grundsätzlich durch das bei der Reaktion der Isocyanatgruppen mit Wasser gebildete Kohlendioxid erfolgen, die Verwendung von weiteren Treibmitteln ist jedoch ebenfalls möglich. So können prinzipiell auch Treibmittel aus der Klasse der Kohlenwasserstoffe wie $C_3$-$C_6$-Alkane, z.B. Butane, _n_-Pentan, _iso_-Pentan, _cyclo_-Pentan, Hexane o.ä. oder halogenierte Kohlenwasserstoffe wie Dichlormethan, Dichlormonofluormethan, Chlordifluorethane, 1,1-Dichlor-2,2,2-Trifluorethan, 2,2-Dichlor-2-fluorethan, insbesondere chlorfreie Fluorkohlenwasserstoffe wie Difluormethan, Trifluormethan, Difluorethan, 1,1,1,2-Tetrafluorethan, Tetrafluorethan (R 134 oder R 134a), 1,1,1,3,3-Pentafluorpropan (R 245 fa), 1,1,1,3,3,3-Hexafluorpropan (R 256), 1,1,1,3,3-Pentafluorbutan (R 365 mfc), Heptafluorpropan oder auch Schwefelhexafluorid verwendet werden. Auch Gemische dieser Treibmittel sind verwendbar.

Bevorzugt beträgt der Ethylenoxidanteil von A2) $\geq$ 50 Gew.-%, oder bevorzugt $\geq$ 55 Gew.-%, oder bevorzugt $\geq$ 60 Gew.-%.

Bevorzugt weist die Mischung der isocyanathaltigen Komponenten ein molares Verhältnis von Urethangruppen zu Isocyanatgruppen in einem Bereich von 1,0 bis 5,0, oder bevorzugt in einem Bereich von 1,1-4,0, oder bevorzugt in einem Bereich von 1,2 bis 3 auf.

Bevorzugt liegt bei der Umsetzung der Komponenten A1) bis A3) zu dem isocyanatfunktionellen Präpolymer A) ein molares Verhältnis von NCO zu OH-Gruppen von < 5, oder bevorzugt von < 4, oder bevorzugt von < 3, oder bevorzugt in einem Bereich von 1 bis 5, oder bevorzugt in einem Bereich von 1,5 bis 4,5 vor.

Bevorzugt weist das isocyanatfunktionelle Präpolymer A) eine Viskosität $\leq$ 50000 mPas, bevorzugt von $\leq$ 25000, oder bevorzugt von $\leq$ 10000, oder bevorzugt in einem Bereich von 100 bis 20000 mPas auf.

Bevorzugt weist mindestens ein Teil der isocyanathaltigen Komponenten eine Isocyanatfunktionalität von > 2, bevorzugt in einem Bereich von 2 bis 6, oder bevorzugt in einem Bereich von 2, 1 bis 5 auf.

Bevorzugt weist das Polyalkylenoxid A2) eine OH-Zahl in einem Bereich von 40 bis 450 mg KOH/g, bevorzugt in einem Bereich von 75 bis 400, oder bevorzugt in einem Bereich von 113 bis 300 auf.

In einer bevorzugten Ausführungsform des Hygieneartikels weist dieser mindestens eine, besonders bevorzugt mindestens zwei und ganz besonders bevorzugt alle der folgenden Eigenschaften auf:

  a. das Verhältnis des Volumens der Absorptionsschicht zu dem Volumen der Speicherschicht in einem Bereich von 1:1 bis 1:50 liegt, bevorzugt in einem Bereich von 1:1,5 bis 1:20, oder bevorzugt in einem Bereich von 1:2 bis 1:10;

  b. die Speicherschicht eine Dicke in einem Bereich von 0,1 bis 20 mm, oder bevorzugt in einem Bereich von 0,35 bis 10 mm, oder bevorzugt in einem Bereich von 0,5 bis 5 mm aufweist;

  c. die Speicherschicht ein maximales Absorptionsvermögen für Wasser von $\geq$ 1000%, oder bevorzugt von $\geq$ 1200%, oder bevorzugt von $\geq$ 1500% aufweist (jeweils g/g nach DIN EN 13726-1:2002);

  d. die Speicherschicht eine Retention von $\geq$ 30%, bevorzugt $\geq$ 40% aufweist, ermittelt nach maximaler Absorption gemäß DIN EN 13726-1:2002 durch Belastung mit 6 kg Gewicht über 20 s auf einem Schaumstück dessen Maße in trockenem Zustand 5 x 5 cm$^2$ betragen,

  e. die Absorptionsschicht eine Dicke in einem Bereich von 0,05 bis 5 mm aufweist, oder bevorzugt in einem Bereich 0,2 bis 3 mm, oder bevorzugt in einem Bereich von 0,5 bis 2,5 mm;

  f. die Absorptionsschicht eine Feuchtigkeitsaufnahmegeschwindigkeit (Wicking) für einen Tropfen Testflüssigkeit von 30 $\mu$l von < 2 s, bevorzugt < 1,5 s, oder bevorzugt < 1 s aufweist;

  g. der Hygieneartikel eine Erhöhung der Flächenausdehnung bei Kontakt mit Wasser in einem Bereich von $\geq$ 0,1 % bis $\leq$ 40 %, oder bevorzugt in einem Bereich von $\geq$ 0,5 % bis $\leq$ 38 %, oder bevorzugt in einem Bereich von $\geq$ 1 % bis $\leq$ 36 %im Vergleich zur ursprünglichen Flächenausdehnung aufweist;

  h. die Speicher- und die Absorptionsschicht untrennbar miteinander verbunden sind; wobei unter untrennbar verstanden wird, dass mindestens eine der beiden Schichten bei dem Versuch sie zu trennen zumindest zum Teil zerstört wird;

  i. die Speicherschicht, die Absorptionsschicht oder der Hygieneartikel bei einer Absorptionsprüfung nach DIN EN 13726-1:2002 eine Volumenquellung von $\leq$ 170 %, bevorzugt von $\leq$ 150 %, oder bevorzugt von $\leq$ 130 %, oder aufweist, bezogen auf das Volumen vor dem Kontakt mit dem Quellungsmittel.

Bevorzugt weist der Hygieneartikel mindestens die Eigenschaften a., b., e., f. und h. auf. Ganz besonders bevorzugt weist der Hygieneartikel mindestens die Eigenschaften a., b., d., e., f., g. und h. auf.

Bevorzugt weist der Hygieneartikel ein Verhältnis von Höhe zu Breite oder Länge, wobei Breite und Länge zusammen dessen Flächenausdehnung ergeben, in einem Bereich von 1:10 bis 1:10000, oder bevorzugt in einem Bereich von 1:50 bis 1:5000, oder bevorzugt in einem Bereich von 1:100 bis 1:2000 auf. Bevorzugt sind die Absorptionsschicht und die Speicherschicht in dem Hygieneartikel eben ausgestaltet und mit ihrer ebenen Fläche miteinander verbunden.

Als Quellung gemäß der Erfindung wird die Ausdehnung des Polyurethanschaumes oder des Schaumverbun-

9

des der Speicherschicht, der Absorptionsschicht oder des Hygieneartikels in mindestens eine Raumrichtung, bevorzugt in der horizontalen Ebene verstanden. Je nachdem, wie der Polyurethanschaum oder der Schaumverbund geformt ist oder welche Umgebung er aufweist, kann die Quellung in alle Raumrichtungen erfolgen, dann kann sie auch als Volumenquellung bezeichnet werden. Bei der Angabe der Quellung in %, wird diese nach Quellung des Schaums in einem Quellungsmittel, wie Wasser oder Salzlösung gemäß DIN EN 13726-1:2002, bestimmt und in Beziehung zu dem ursprünglichen Wert des Polyurethanschaum oder des Schaumverbund vor dem Kontakt mit dem Quellungsmittel in einer vorbestimmten Raumrichtung gesetzt. Da bei der Bestimmung der Quellung bevorzugt ein Körper quadratischer Grundfläche (bezogen auf die horizontale Ebene) des Polyurethanschaumes bzw. des Schaumverbundes verwendet wird, ist die Ausdehnung entlang einer Kante des Testkörpers ein Maß für die Quellung einer Fläche in der horizontalen Ebene. Daher wird im Folgenden äquivalent zu dem Begriff der Quellung auch der Begriff der Längsausdehnung verwendet. Bevorzugt dehnt sich der Polyurethanschaum oder der Schaumverbund in alle Raumrichtungen gleichmäßig aus. Alternativ kann die Quellung in mindestens einer der drei Raumrichtungen verschieden von mindestens einer weiteren Raumrichtung sein, insbesondere hervorgerufen durch die Schwerkraft.

Bevorzugt ist die Polyurethandispersion a) erhältlich, indem wenigstens die folgenden Komponenten umgesetzt werden:

a1 ein isocyanatfunktionelles Prepolymer,

a2) gegebenenfalls ein isocyanatreaktives, bevorzugt aminofunktionelles, anionisches oder potentiell anionisches Hydrophilierungsmittel,

a3) gegebenenfalls eine aminofunktionelle Verbindung, insbesondere mit einem Molekulargewicht von 32 bis 400 g/mol,

a4) Wasser,

wobei das Hydrophilierungsmittel a2) dann obligatorisch ist, wenn das Prepolymer a1) keine hydrophilierenden Gruppen aufweist.

In einer bevorzugten Ausführungsform des Hygieneartikels ist die Polyurethandispersion a) erhältlich, indem

A). isocyanatfunktionelle Prepolymere aus

A1). organischen Polyisocyanaten

A2). polymeren Polyolen mit zahlenmittleren Molekulargewichten von 400 bis 8000 g/mol und OH-Funktionalitäten von 1,5 bis 6 und

A3). gegebenenfalls hydroxyfunktionellen Verbindungen mit Molekulargewichten von 62 bis 399 g/mol und

A4). gegebenenfalls isocyanatreaktiven, anionischen oder potentiell anionischen und gegebenenfalls nichtionischen Hydrophilierungsmitteln,

hergestellt, und

B). deren freie NCO-Gruppen dann ganz oder teilweise

B1). gegebenenfalls mit aminofunktionellen Verbindungen mit Molekulargewichten von 32 bis 400 g/mol und

B2). mit aminofunktionellen, anionischen oder potentiell anionischen Hydrophilierungsmitteln

unter Kettenverlängerung umgesetzt werden und die Prepolymere vor, während oder nach Schritt B). in Wasser dispergiert werden. Die so erhaltene Polyurethandispersion wird im Folgenden als Polyurethan-Dispersion (A) bezeichnet.

In einer alternativen Ausgestaltung des Hygieneartikels ist die Polyurethandispersion a) erhältlich durch reaktive Umsetzung wenigstens folgender Komponenten:

A. einer aliphatischen Polyisocyanat-Komponente mit einer mittleren Isocyanatfunktionalität von $\geq$ 1,8 und $\leq$ 2,6,

B. einer polymeren Polyether-Polyol-Komponente;

C. einer aminofunktionellen Kettenverlängerer-Komponente mit mindestens 2 isocyanatreaktiven Aminogruppen, enthaltend wenigstens eine aminofunktionelle Verbindung C1., die keine ionischen oder ionogenen Gruppen aufweist und/oder eine aminofunktionelle Verbindung C2., die anionische oder anionogene Gruppen aufweist,

D. gegebenenfalls weiteren hydrophilierenden Komponenten, die unterschiedlich sind von C2.,

E. gegebenenfalls hydroxyfunktionellen Verbindungen mit einem Molekulargewicht von 62 bis 399 mol/g,

F. gegebenenfalls weiteren polymeren Polyolen, welche unterschiedlich sind von B.,

G. einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und

H. optional einer aliphatischen Polyisocyanat-Komponente mit einer mittleren Isocyanatfunktionalität von > 2,6 und ≤ 4,

wobei die Komponenten B. und F. zusammen ≤ 30 Gew.-% an Komponente F., bevorzugt ≤ 20 Gew.-% an Komponente F., oder bevorzugt ≤ 10 Gew.-% an Komponente F., bezogen auf die Gesamtmasse der Komponenten B. und F. enthalten.

Die auf diese Weise durch reaktive Umsetzung erhaltene Polyurethandispersion a) wird im Folgenden als Polyurethan-Dispersion (B) bezeichnet.

In einer weiteren bevorzugten Ausführungsform des Hygieneartikels beinhaltet die Polyurethandispersion a) sowohl die Polyurethan-Dispersion (A) als auch die Polyurethan-Dispersion (B), wobei sich die beiden Dispersionen (A) und (B) in mindestens einer Eigenschaft unterschieden.

Die Polyurethan-Dispersionen (A) und (B) unterscheiden sich bevorzugt in ihrer Zusammensetzung mindestens in der Auswahl oder Menge mindestens einer Komponente. Polyurethan-Dispersionen (A) und (B) sind folglich bevorzugt mindestens in einem Teil des jeweiligen Polymers chemisch verschieden und voneinander unterscheidbar.

Bevorzugt sind die Bestandteile der Dispersionen (A) und (B) sowie alle weiteren Komponenten zur Bildung des Hygieneartikels gering bis nicht-zytotoxisch. Unter gering bis nicht-zytotoxisch gemäß der Erfindung wird verstanden, dass der Hygieneartikel und bevorzugt sämtliche Bestandteile des Hygieneartikels eine Viabilität von ≥ 70 %, bevorzugt ≥ 80 %, beziehungsweise eine Klassifizierung von 0 bis 2 ("no to mild", was keine bis geringe Zytotoxizität bedeutet) in einem Zytotoxtest nach DIN ISO 10993-5:2009-10 aufweisen.

Bevorzugt liegt das Gewichts-Verhältnis der Polyurethan-Dispersion (A) zu der Polyurethan-Dispersion (B) in der Polyurethandispersion a) in einem Bereich von 1:1 bis 5:1, bevorzugt in einem Bereich von 1:1 bis 4:1, oder bevorzugt in einem Bereich von 1:1 bis 3:1, bezogen auf die Gesamtmasse aus den Massen der Dispersionen (A) und (B) in der Polyurethandispersion a). Bevorzugt weist ein durch Trocknung aus der Polyurethan-Dispersion (B) gebildeter Film eine Bruchspannung von ≤ 5 MPa, bevorzugt ≤ 3,5 MPa und besonders bevorzugt ≤ 2,5 MPa. Der aus der Dispersion (B) gebildete Film weist bevorzugt eine Bruchdehnung von ≥ 1750 %, bevorzugt ≥ 2000 %, bevorzugt in einem Bereich von ≥ 1750 bis 3 000 %, besonders bevorzugt von 2000 bis 4000 % auf. Bevorzugt ist der 500 % Modul des aus Dispersion (B) gebildeten Films bei bevorzugt ≤ 2 MPa, oder bevorzugt ≤ 1,5 MPa oder bevorzugt ≤ 1,0 MPa, besonders bevorzugt in einem Bereich von 0,1 bis 2 MPa. Bevorzugt ist der Polyurethanharnstoff, gebildet aus der Polyurethan-Dispersion (B), amorph und weist einen Tg ≤ - 25 °C, bevorzugt ≤ - 40 °C, oder bevorzugt ≤ - 50 °C auf, bestimmt mittels dynamischer Differenzkalorimetrie gemäß DIN EN 61006, Verfahren A.

Ein Film gebildet aus der Polyurethan-Dispersion (A) weist bevorzugt eine Bruchdehnung im Bereich von 400 % bis 700 %, eine Bruchspannung von 20 bis 50 MPa und einen 100% Modul von bevorzugt ≤ 7 MPa, bevorzugt in einem Bereich von 2 bis 7 MPa auf. Das Verfahren zur Herstellung des Films aus Dispersion (A) wird später im Detail beschrieben.

Bevorzugt weist ein aus der Polyurethan-Dispersion (A) gebildeter Film eine Bruchdehnung auf, die in einem Bereich von 1 bis 50 % der Bruchdehnung eines aus der Polyurethan-Dispersion (B) gebildeten Films liegt. Bevorzugt weist ein aus der Polyurethan-Dispersion (A) gebildeter Film eine Bruchspannung auf, die in einem Bereich von 5 bis 40 % der Bruchspannung eines aus der Polyurethan-Dispersion (B) gebildeten Films liegt. Bevorzugt weist ein aus der Polyurethan-Dispersion (A) gebildeter Film einen 100% Modul auf, das in einem Bereich von 10 bis 50 % des 100% Moduls eines aus der Polyurethan-Dispersion (B) gebildeten Films liegt.

Polyurethan-Dispersion (A)

Um bei der Herstellung der Polyurethan-Dispersion (A) eine anionische Hydrophilierung zu erreichen, werden in A4). und/oder B2). bevorzugt Hydrophilierungsmittel eingesetzt, die wenigstens eine gegenüber NCO-Gruppen reaktive Gruppe wie im Falle von A4). Amino-, Hydroxy- oder Thiolgruppen und im Falle von B2). Aminogruppen aufweisen und darüber hinaus -COO- oder -SO$_3$-oder -PO$_3$$^{2-}$ als anionische bzw. deren ganz oder teilweise protonierte Säureformen als potentiell anionische Gruppen aufweisen.

Bevorzugte wässrige, anionische Polyurethan-Dispersionen (A) haben einen niedrigen Grad an hydrophilen anionischen Gruppen, bevorzugt von 2 bis 200 Milliäquivalenten, besonders bevorzugt von weniger als 10 bis 100 Milliäquivalenten pro 100 g Festharz.

Um eine gute Sedimentationsstabilität zu erreichen, liegt die zahlenmittlere Teilchengröße der Polyurethan-Dispersionen (A) bevorzugt bei weniger als 750 nm, besonders bevorzugt bei weniger als 500 nm, bestimmt mittels Laserkorrelations-Spektroskopie.

Das Verhältnis von NCO-Gruppen der Verbindungen aus Komponente A1). zu NCO-reaktiven Gruppen wie Amino-, Hydroxy- oder Thiolgruppen der Verbindungen der Komponenten A2). bis A4). beträgt bei der Herstellung des NCO-funktionellen Prepolymers 1,05 bis 3,5; bevorzugt 1,2 bis 3,0 und besonders bevorzugt 1,3 bis 2,5.

Die aminofunktionellen Verbindungen in Stufe B) werden in solch einer Menge eingesetzt, dass das Äquivalentverhältnis von isocyanatreaktiven Aminogruppen dieser Verbindungen zu den freien Isocyanatgruppen des Prepolymers 40 bis 150 %, bevorzugt 50 bis 125 %, besonders bevorzugt 60 bis 100 % beträgt.

Geeignete Polyisocyanate der Komponente A1). sind die dem Fachmann an sich bekannten aromatischen, araliphatischen, aliphatischen oder cycloaliphatischen Polyisocyanate einer NCO-Funktionalität von $\geq$ 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis-(isocyanatomethyl)benzol (XDI), sowie Alkyl-2,6-diisocyanatohexanoate (Lysindiisocyanate) mit C1-C8-Alkylgruppen.

Neben den vorstehend genannten Polyisocyanaten können anteilig auch modifizierte Diisocyanate mit Uretdion-, Isocyanurat-, Urethan-, Allophanat-, Biuret-, Iminooxadiazindion- und/oder Oxadiazintrionstruktur sowie nicht-modifiziertes Polyisocyanat mit mehr als 2 NCO-Gruppen pro Molekül wie z.B. 4-Isocyanatomethyl-1,8-octandiisocyanat (Nonantriisocyanat) oder Triphenylmethan-4,4',4''-triisocyanat mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugt 2 bis 2,6 und besonders bevorzugt 2 bis 2,4.

Besonders bevorzugt werden in A1). 1,6-Hexamethylendiisocyanat, Isophorondiisocyanat, die isomeren Bis(4,4'-isocyanatocyclohexyl)methane, sowie deren Mischungen eingesetzt.

In A2). werden polymere Polyole mit einem zahlenmittleren Molekulargewicht Mn von 400 bis 8000 g/mol, bevorzugt von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2). einzeln oder in beliebigen Mischungen untereinander eingesetzt werden.

Solche Polyesterpolyole sind die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, 1,3-Butandiol, 1,4-Butandiol, 1,6-Hexandiol und Isomere, Neopentylglykol oder Hydroxypivalinsäureneopentylglykolester, wobei 1,6-Hexandiol und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton. Ebenfalls können in A2). Hydroxylgruppen aufweisende Polycarbonate, bevorzugt Polycarbonatdiole, mit zahlenmittleren Molekulargewichten Mn von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonatdiol 40 bis 100 Gew.-% Hexandiol, bevorzugt 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2). eingesetzt werden. Die Hydroxylgruppen aufweisenden Polycarbonate sind bevorzugt linear gebaut.

Ebenfalls können in A2). Polyetherpolyole eingesetzt werden. Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrins an di- oder polyfunktionelle Startermoleküle. Polyetherpolyole, basierend auf der zumindest anteiligen Addition von Ethylenoxid an di- oder polyfunktionelle Startermoleküle, können auch als Komponente A4). eingesetzt werden (nichtionische Hydrophilierungsmittel).

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

Besonders bevorzugte Ausführungsformen der Polyurethan-Dispersionen (A) enthalten als Komponente A2). eine Mischung aus Polycarbonatpolyolen und Polytetramethylenglykolpolyolen, wobei in dieser Mischung der Anteil an Polycarbonatpolyolen in der Mischung 0 bis 80 Gew.-% und der Anteil an Polytetramethylenglykolpolyolen 100 bis 20 Gew.-% beträgt. Bevorzugt ist ein Anteil von 50 bis 100 Gew.-% an Polytetramethylenglykolpolyolen und ein Anteil von 0 bis 50 Gew.-% an Polycarbonatpolyolen. Besonders bevorzugt ist ein Anteil von 75 bis 100 Gew.-% an Polytetramethylenglykolpolyolen und ein Anteil von 0 bis 25 Gew.-% an Polycarbonatpolyolen, jeweils mit der Maßgabe, dass die Summe der Gewichtsprozente der Polycarbonat- und Polytetramethylenglykolpolyole 100 % ergibt und der Anteil der Summe der Polycarbonat- und Polytetramethylenglykolpolyetherpolyole an der Komponente A2). mindestens 50 Gew.-%, bevorzugt 60 Gew.-% und besonders bevorzugt mindestens 70 Gew.-% beträgt.

Die Verbindungen der Komponente A3). besitzen Molekulargewichte von 62 bis 400 g/mol.

In A3). können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie $\alpha$-Hydroxybutyl-$\epsilon$-hydroxycapronsäureester, $\omega$-Hydroxyhexyl-$\gamma$-hydroxybuttersäure-ester, Adipinsäure-($\beta$-hydroxyethyl)ester oder Terephthalsäure-bis($\beta$-hydroxyethyl)-ester.

Ferner können in A3). auch monofunktionelle, isocyanatreaktive, hydroxylgruppenhaltige Verbindungen eingesetzt werden. Beispiele solcher monofunktionellen Verbindungen sind Ethanol, n-Butanol, Ethylenglykolmonobutylether, Diethylenglykolmonomethylether, Ethylenglykolmonobutylether, Diethylenglykolmonobutylether, Propylenglykolmonomethylether, Dipropylenglykolmonomethylether, Tripropylenglykolmonomethylether, Dipropylenglykolmonopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol. Bevorzugte Verbindungen der Komponente A3). sind 1,6-Hexandiol. 1,4-Butandiol, Neopentylglykol und Trimethylolpropan.

Unter anionisch bzw. potentiell anionisch hydrophilierenden Verbindungen der Komponente A4). werden sämtliche Verbindungen verstanden, die mindestens eine isocyanatreaktive Gruppe wie eine Hydroxylgruppe aufweisen sowie mindestens eine Funktionalität wie z.B. $-COO^-M^+$, $-SO_3^-M^+$, $-PO(O^-M^+)_2$ mit $M^+$ beispielsweise gleich Metallkation, $H^+$, $NH_4^+$, $NHR_3^+$, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, Mono- und Dihydroxysulfonsäuren sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und $NaHSO_3$, wie es in DE-A 2 446 440, Seite 5-9, Formel I-III beschrieben ist. Bevorzugte anionische oder potentiell anionische Hydrophilierungsmittel der Komponente A4). sind solche der vorstehend genannten Art, die über Carboxylat- bzw Carbonsäuregruppen und/oder Sulfonatgruppen verfügen.

Besonders bevorzugte anionische oder potentiell anionische Hydrophilierungsmittel sind solche, die Carboxylat- bzw. Carbonsäuregruppen als ionische oder potentiell ionische Gruppen enthalten, wie Dimethylolpropionsäure, Dimethylolbuttersäure und Hydroxypivalinsäure bzw. deren Salze.

Geeignete nichtionisch hydrophilierende Verbindungen der Komponente A4). sind z.B. Polyoxyalkylenether, die mindestens eine Hydroxy- oder Aminogruppe, bevorzugt mindestens eine Hydroxygruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38).

Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Bevorzugte Polyethylenoxidether der vorstehend genannten Art sind monofunktionelle gemischte Polyalkylenoxidpolyether, die 40 bis 100 mol-% Ethylenoxid- und 0 bis 60 mol-% Propylenoxideinheiten aufweisen.

Bevorzugte nichtionisch hydrophilierende Verbindungen der Komponente A4). sind solche der vorstehend genannten Art, wobei es sich um Block(co)polymere handelt, die durch blockweise Addition von Alkylenoxiden an geeignete Starter hergestellt werden.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Als Komponente B1). können Di- oder Polyamine wie 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Triaminononan, 1,3- und 1,4-Xylylendiamin, $\alpha,\alpha,\alpha',\alpha'$-Tetramethyl-1,3- und -1,4-xylylendiamin und 4,4-Diaminodicyclohexylmethan und/oder Dimethylethylendiamin eingesetzt werden. Ebenfalls möglich, aber weniger bevorzugt, ist die Verwendung von Hydrazin sowie Hydraziden wie Adipinsäuredihydrazid.

Darüber hinaus können als Komponente B1). auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclo-hexylaminopropan, 3-Amino-1-Methylaminobutan, Alkanolamine wie N-Aminoethylethanolamin, Ethanolamin, 3-Aminopropanol, Neopentanolamin.

Ferner können als Komponente B1). auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin.

Bevorzugte Verbindungen der Komponente B1). sind 1,2-Ethylendiamin, 1,4-Diaminobutan und Isophorondiamin. Besonders bevorzugt werden Mischungen der vorgenannten Diamine der Komponente B1). eingesetzt, insbesondere Mischungen aus 1,2-Ethylendiamin und Isophorondiamin sowie Mischungen aus 1,4-Diaminobutan und Isophorondiamin.

Unter anionisch bzw. potentiell anionisch hydrophilierenden Verbindungen der Komponente B2). werden sämtliche Verbindungen verstanden, die mindestens eine Amino-Gruppe aufweisen, sowie mindestens eine Funktionalität wie z.B. $-COO^-M^+$, $-SO_3^-M^+$, $-PO(O^-M^+)_2$ mit $M^+$ beispielsweise gleich Metallkation, $H^+$, $NH_4^+$, $NHR_3^+$, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann.

Geeignete anionisch oder potentiell anionisch hydrophilierende Verbindungen sind Mono- und Diaminocarbonsäuren, Mono- und Diaminosulfonsäuren sowie Mono- und Diaminophosphonsäuren und ihre Salze. Beispiele solcher anionischen bzw. potentiell anionischen Hydrophilierungsmittel sind N-(2-Aminoethyl)-β-alanin, 2-(2-Amino-ethylamino)-ethansulfonsäure, Ethylendiamin-propyl- oder - butylsulfonsäure, 1,2- oder 1,3-Propylendiamin-β-ethylsulfonsäure, Glycin, Alanin, Taurin, Lysin, 3,5-Diaminobenzoesäure und das Additionsprodukt von IPDA und Acrylsäure (EP-A 0 916 647, Beispiel 1). Weiterhin kann Cyclohexylaminopropansulfonsäure (CAPS) aus WO-A 01/88006 als anionisches oder potentiell anionisches Hydrophilierungsmittel verwendet werden.

Zur Hydrophilierung können auch Mischungen aus anionischen bzw. potentiell anionischen Hydrophilierungsmitteln und nichtionischen Hydrophilierungsmitteln verwendet werden.

In einer bevorzugten Ausführungsform zur Herstellung der speziellen Polyurethan-Dispersionen (A) werden die Komponenten A1). bis A4). und B1). bis B2). in den folgenden Mengen eingesetzt, wobei sich die Einzelmengen stets zu 100 Gew.-% addieren:

5 bis 40 Gew.-%, besonders bevorzugt 5 bis 35 Gew.-% Komponente A1).,

55 bis 90 Gew.-%, besonders bevorzugt 60 bis 90 A2).,

0 bis 20 Gew.-%, besonders bevorzugt 0,5 bis 15 Gew.-% Summe der Komponenten A3). und B1).

0,1 bis 25 Gew.-%, besonders bevorzugt 0,5 bis 15 Gew.-% Summe der Komponenten A4). und B2)., wobei bezogen auf die Gesamtmengen der Komponenten A1). bis A4). und B1). bis B2). 0,1 bis 10 Gew.-% an anionischen bzw. potentiell anionischen Hydrophilierungsmitteln aus A4). und/oder B2). verwendet werden.

Die Herstellung der anionisch hydrophilierten Polyurethan-Dispersionen (A) kann in einer oder mehreren Stufe/-n in homogener oder bei mehrstufiger Umsetzung, teilweise in disperser Phase durchgeführt werden. Nach vollständig oder teilweise durchgeführter Polyaddition aus A1). bis A4). erfolgt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z.B. Prepolymer-Mischverfahren, Acetonverfahren oder Schmelzdispergierverfahren verwendet werden. Bevorzugt wird das Aceton-Verfahren verwendet.

Für die Herstellung nach dem Aceton-Verfahren werden üblicherweise die Bestandteile A2). bis A4). und die Polyisocyanatkomponente A1). zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanataddtitionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von N-Methylpyrrolidon, N-Ethylpyrrolidon in der Dispersion verbleiben. Bevorzugt werden aber außer den genannten aliphatischen, ketofunktionellen Lösemitteln keine anderen Lösungsmittel verwendet.

Anschließend werden die gegebenenfalls zu Beginn der Reaktion noch nicht zugegebenen Bestandteile von A1). bis A4). zudosiert.

Bei der Herstellung des Polyurethan-Prepolymeren aus A1). bis A4). beträgt das Stoffmengenverhältnis von Isocyanatgruppen zu mit isocyanatreaktiven Gruppen 1,05 bis 3,5; bevorzugt 1,2 bis 3,0 und besonders bevorzugt 1,3 bis 2,5.

Die Umsetzung der Komponenten A1). bis A4). zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-Methylmorpholin, Methyldiisopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt zwischen 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, indem das Dispergierwasser bereits das Neutralisationsmittel enthält.

Im Anschluss wird in einem weiteren Verfahrensschritt, falls noch nicht oder nur teilweise geschehen das erhaltene Prepolymer mit Hilfe von aliphatischen Ketonen wie Aceton oder 2-Butanon gelöst.

Bei der Kettenverlängerung in Stufe B) werden $NH_2$- und/oder NH-funktionelle Komponenten mit den noch verbliebenen Isocyanatgruppen des Prepolymers teilweise oder vollständig umgesetzt. Bevorzugt wird die Kettenverlängerung/-terminierung vor der Dispergierung in Wasser durchgeführt.

Zur Kettenterminierung werden üblicherweise Amine B1). mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionische oder potentiell anionische Hydrophilierungsmittel entsprechend der Definition B2). mit $NH_2$- oder NH-Gruppen eingesetzt, erfolgt die Kettenverlängerung der Prepolymere bevorzugt vor der Dispergierung.

Die aminischen Komponenten B1). und B2). können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den Polyurethan-Dispersionen (A) beträgt typischerweise weniger als 1,0 Gew.-%, bevorzugt weniger als 0,5 Gew.-%, bezogen auf die gesamte Dispersion.

Der pH-Wert der erfindungswesentlichen Polyurethan-Dispersionen (A) beträgt typischerweise weniger als 9,0, bevorzugt weniger als 8,5; besonders bevorzugt weniger als 8,0 und liegt ganz besonders bevorzugt bei 6,0 bis 7,5.

Der Feststoffgehalt der Polyurethan-Dispersionen (A) beträgt bevorzugt 35 bis 70 Gew.-%, besonders bevorzugt 40 bis 65 Gew.-%, ganz besonders bevorzugt 45 bis 60 Gew.-% und insbesondere 45 bis 55 Gew.-%.

Die Menge an anionischen oder potentiell anionischen Gruppen auf der Partikeloberfläche, gemessen über eine Säure-Base-Titration liegt im Allgemeinen bei 2 bis 500 mmol, bevorzugt 30 bis 400 mmol pro 100 Gramm Festkörper.

Polyurethan-Dispersion (B)

Bei der Herstellung der Polyurethan-Dispersion (B) wird zunächst ein isocyanatgruppenhaltiges Prepolymer gebildet, das mit Aminogruppen Kettenverlängert wird. Aufgrund der Verwendung von Aminogruppen-haltigen Verbindungen bei der Herstellung der Polyurethan-Dispersion (B) können auch Harnstoffgruppen entstehen, warum im Folgenden das Polyurethan, das in der Polyurethan-Dispersion (B) enthalten ist auch Polyurethanharnstoff genannt wird und daher die Polyurethan-Dispersion (B) auch als Polyurethanharnstoff-Dispersion (B) bezeichnet wird.

Unter ionogenen Gruppen werden im Zusammenhang mit der Polyurethanharnstoff-Dispersion (B) solche funktionellen Gruppen verstanden, die in der Lage sind, beispielsweise durch Neutralisation mit einer Base, ionische Gruppen zu bilden.

Die Komponente A. kann jedes Polyisocyanat sein, das der Fachmann hierfür verwenden würde. Als Komponente A. bevorzugt geeignete Polyisocyanate sind insbesondere die dem Fachmann an sich bekannten aliphatischen Polyisocyanate mit einer mittleren Isocyanat-funktionalität von ≥ 1,8 und ≤ 2,6. Der Begriff aliphatisch umfasst dabei auch cycloaliphatische und/oder araliphatische Polyisocyanate.

Unter der mittleren Isocyanatfunktionalität wird dabei die durchschnittliche Anzahl an Isocyanatgruppen pro Molekül verstanden.

Bevorzugte Polyisocyanate sind solche des Molekulargewichtsbereichs von 140 bis 336 g/mol. Besonders bevorzugt sind diese ausgewählt aus der Gruppe bestehend aus. 1,4-Diisocyanatobutan (BDI), 1,5-Pentandiisocyanat, (PDI) 1,6-Diisocyanatohexan (HDI), 1,3-Bis(isocyanatomethyl)benzol (1,3-Xylylendiisocyanat, XDI), 1,4-Bis(isocyanatomethyl)benzol (1,4-Xylylendiisocyanat, XDI), 1,3-Bis(1-isocyanato-1-methyl-ethyl)benzol (TMXDI), 1,4-Bis(1-isocyanato-1-methyl-ethyl)benzol (TMXDI), 4-Isocyanatomethyl-1,8-octandiisocyanat (Trisisocyanatononan (TIN)), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan sowie die cycloaliphatischen Diisocyanate 1,3- bzw. 1,4-Diisocyanatocyclohexan, 1,4-Di¬isocyanato-3,3,5-trimethylcyclohexan, 1,3-Diisocyanato-2(4)-methylcyclohexan, 1-Isocyanato-3,3,5-trimethyl-5-isocyanatomethyl-cyclohexan (Isophorondiisocyanat, IPDI), 1-Isocyanato-1-methyl-4(3)isocyanato-me-thylcyclohexan, 1,8-Diisocyanato-p-menthan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetramethyl-1,1'-bi(cyclohexyl), 4,4' und/oder 2,4'-Diisocyanatodicyclohexylmethan, 4,4' Diisocyanato-3,3'-dimethyldicyclohexylmethan, 4,4' Diisocyanato-3,3',5,5'-tetramethyldicyclohexylmethan, 1,3-Diisocyanatoadamantan, und 1,3-Dimethyl-5,7-diisocyanatoadamantan oder beliebige Gemische solcher Isocyanate. Ganz besonders bevorzugt sind die Polyisocyanate aus-gewählt aus 1,4-Butylendiisocyanat, 1,5-Pentamethylendiisocyanat (PDI), 1,6-Hexamethy-lendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)methane oder deren Mischungen beliebigen Isomerengehalts (H12-MDI), 1,4-Cyclohexylendiisocyanat, 4-Isocyanatomethyl-1,8-octandiisocyanat (Nonantriisocyanat) sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate, die eine mittlere Isocyanatfunktionalität ≥ 2 und ≤ 2,6 aufweisen, mit Uretdion-, Isocyanu-rat-, Urethan-, Allophanat-, Biuret-, Iminooxadiazindion- oder Oxadiazintrionstruktur sowie Mischungen aus diesen und/oder den oben stehenden anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocya-natgruppen oder Mischungen aus diesen und einer mittleren NCO-Funktionalität der Mi-schung von > 1,8 und ≤ 2,6 und besonders bevorzugt ≥ 2,0 und ≤ 2,4.

Besonders bevorzugt enthält die organische Polyisocyanat-Komponente A. ein aliphatisches oder cycloaliphatisches Polyisocyanat ausgewählt aus HDI, IPDI und/oder H12-MDI oder deren Modifikationsprodukten, ganz besonders bevorzugt ausgewählt aus HDI und/oder IPDI.

In einer bevorzugten Variante liegen als Komponente A. IPDI und HDI im Gemisch vor.

Das Gewichtsverhältnis von IPDI:HDI liegt dabei bevorzugt im Bereich von 1,05 bis 10, besonders bevorzugt im Bereich von 1,1 bis 5, und ganz besonders bevorzugt im Bereich von 1,1 bis 1,5.

Bevorzugt wird zur Herstellung des erfindungsgemäß verwendeten Polyurethanharnstoffs ≥ 5 und ≤ 40 Gew.-% der Komponente A. und besonders bevorzugt ≥ 10 und ≤ 35 Gew.-% der Komponente A., jeweils bezogen auf die Gesamtmasse des Polyurethanharnstoffs, eingesetzt.

Bevorzug wird zur Herstellung des Polyurethanharnstoffs auch die Komponente H., eine aliphatische Polyisocyanat-Komponente mit einer mittleren Isocyanatfunktionalität (mittlere Anzahl Isocyanatgruppen pro Molekül) von > 2,6 und ≤ 4, vorzugsweise ≥ 2,8 und ≤ 3,8 eingesetzt. Die Komponente H. wird dabei bevorzugt im Gemisch mit Komponente A. eingesetzt.

Als Komponente H. besonders geeignet sind oligomere Diisocyanate, die eine Funktionalität > 2,6 und ≤ 4, vorzugsweise ≥ 2,8 und ≤ 3,8 aufweisen, mit Isocyanurat-, Urethan-, Allophanat-, Biuret-, Iminooxadiazindion- oder Oxadiazintrionstruktur. Ganz besonders bevorzugt enthält H. Isocyanuratstrukturen.

Bevorzugt besteht die organische Polyisocyanat-Komponente H. aus einem aliphatischen oder cycloaliphatischen Polyisocyanat-Oligomer basierend auf HDI, IPDI und/oder H12-MDI, ganz besonders bevorzugt basierend auf HDI.

Das Molverhältnis der NCO-Gruppen aus Komponente A. zu Komponente H. beträgt dabei bevorzugt 100:0,5 bis 100: 50; besonders bevorzugt 100: 2 bis 100: 15 und ganz besonders bevorzugt 100:3 bis 100: 8.

Bevorzug wird zur Herstellung des erfindungsgemäß verwendeten Polyurethanharnstoffs ≥ 0 und ≤ 10 Gew.-% der Komponente H. und besonders bevorzugt ≥ 0,1 und ≤ 3 Gew.-% der Komponente H., jeweils bezogen auf die Gesamtmasse des Polyurethanharnstoffs, eingesetzt.

Die erfindungsgemäß als Komponente B. eingesetzten polymeren Polyether-Polyole weisen bevorzugt zahlenmittlere Molekulargewichte von ≥ 500 und ≤ 8000 g/mol, bestimmt durch Gelpermeationschromatographie gegenüber Polystyrol-Standard in Tetrahydrofuran bei 23°C, bevorzugter ≥ 400 und ≤ 6000 g/mol und besonders bevorzugt von ≥ 600 und ≤ 3000 g/mol und/oder OH-Funktionalitäten von bevorzugt ≥ 1,5 und ≤ 6, bevorzugter ≥ 1,8 und ≤ 3, besonders bevorzugt von ≥ 1,9 und ≤ 2,1 auf. Der Ausdruck "polymere" Polyether-Polyole bedeutet hier insbesondere, dass die genannten Polyole mindestens drei, bevorzugter mindestens vier miteinander verbundene Wiederholungseinheiten aufweisen.

Das zahlenmittlere Molekulargewicht wird im Rahmen dieser Anmeldung bestimmt durch Gelpermeationschromatographie (GPC) in Tetrahydrofuran bei 23°C, außer wenn dies anders beschrieben wird. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2xPSS SDV linear M, 8x300 mm, 5 μm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.

Geeignete Polyetherpolyole sind beispielsweise die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten. Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

Bevorzug enthält die Komponente B. Poly(tetramethylenglykol)polyetherpolyole oder besteht daraus.

Geeignete Poly(tetramethylenglykol)polyetherpolyole sind beispielsweise durch Polymerisation von Tetrahydrofuran mittels kationischer Ringöffnung erhältlich sind.

Bevorzugt enthält die Komponente B. ein Gemisch aus Poly(tetramethylenglykol)polyetherpolyolen oder besteht daraus, wobei sich die Poly(tetramethylenglykol)polyetherpolyole in ihren zahlenmittleren Molekulargewichten unterscheiden. Bevorzugt enthält die Komponente B. ein Gemisch aus Poly(tetramethylenglykol)polyetherpolyolen I mit einem zahlenmittleren Molekulargewichte $M_n$ in einem Bereich von ≥ 400 und ≤ 1500 g/mol, besonders bevorzugt in einem Bereich von ≥ 600 und ≤ 1200 g/mol, ganz besonders bevorzugt in einem Bereich von 1000 g/mol und Poly(tetramethylenglykol)polyetherpolyole II mit einem zahlenmittleren Molekulargewichte $M_n$ in einem Bereich von ≥ 1500 und ≤ 8000 g/mol, besonders bevorzugt in einem Bereich von ≥ 1800 und ≤ 3000 g/mol, ganz besonders bevorzugt von 2000 g/mol.

Das Gewichtsverhältnis der Poly(tetramethylenglykol)polyetherpolyole I zu den Poly(tetramethylenglykol)polyetherpolyolen II liegt bevorzugt im Bereich von 0,1 bis 10, besonders bevorzugt im Bereich von 0,2 bis 10, ganz besonders bevorzugt im Bereich von 1 bis 6.

Erfindungsgemäß wird zur Herstellung des Polyurethanharnstoffs eine aminofunktionelle Kettenverlängerer-Komponente C. mit mindestens 2 isocyanatreaktiven Aminogruppen, enthaltend wenigstens eine aminofunktionelle Verbindung C1., die keine ionischen oder ionogenen Gruppen aufweist und/oder eine aminofunktionelle Verbindung C2., die ionische oder ionogene Gruppen aufweist, eingesetzt.

Die aminofunktionellen Verbindungen der Komponente C. Komponente werden bevorzugt aus primären und/oder sekundären Diaminen ausgewählt. Insbesondere umfassen die aminofunktionellen Verbindungen C. mindestens ein Diamin.

Bevorzugt umfasst die aminofunktionelle Komponente C. wenigstens eine aminofunktionelle Verbindung C2., die ionische und/oder ionogene Gruppen aufweist.

Bevorzugt umfasst die aminofunktionelle Komponente C. sowohl aminofunktionelle Verbindungen C2., die ionische und/oder ionogene Gruppe aufweisen, als auch aminofunktionelle Verbindungen C1., die keine ionische oder ionogene Gruppe aufweisen.

Beispielsweise können als Komponente C1. organische Di- oder Polyamine wie beispielsweise 1,2-Ethylendiamin, 1,2- und 1,3-Diaminopropan, 1,4-Diaminobutan, 1,6-Diaminohexan, Isophorondiamin (IPDA), Isomerengemisch von 2,2,4- und 2,4,4-Trimethylhexamethylendiamin, 2-Methylpentamethylendiamin, Diethylentriamin, 4,4-Diaminodicyclohexylmethan und/oder Dimethylethylendiamin oder Mischungen aus mindestens zwei hiervon eingesetzt werden.

Bevorzugt ist die Komponente C1. ausgewählt aus der Gruppe bestehend aus 1,2-Ethylendiamin, Bis(4-aminocyclohexyl)methan, 1,4-Diaminobutan, IPDA, Ethanolamin, Diethanolamin und Diethylentriamin oder einer Mischung aus mindestens zwei hiervon.

Bevorzugt enthält die Komponente C1. ≥ 75 mol%, besonders bevorzugt ≥ 80 mol%, ganz besonders bevorzugt ≥ 85 mol%, weiterhin bevorzugt ≥ 95 mol% und des Weiteren bevorzugt 100 mol% 1,2-Ethylendiamin oder IPDA oder ein Gemisch aus 1,2-Ethylendiamin und IPDA, wobei die Summe der beiden Amine in Bezug auf die Gesamtmenge an C1. bevorzugt in den genannten Bereichen vorliegt. Bevorzugt enthält die Komponente C1. ≥ 75 mol%, besonders bevorzugt ≥ 80 mol%, ganz besonders bevorzugt ≥ 85 mol%, weiterhin bevorzugt ≥ 95 mol% und des Weiteren bevorzugt 100 mol% 1,2-Ethylendiamin.

Bevorzugt umfasst die hydrophilierende Komponente C2. mindestens eine anionisch hydrophilierende Verbindung. Weiterhin bevorzugt beinhaltet die hydrophilierende Komponente C2. eine anionisch hydrophilierende Verbindung zu mindestens 80 Gew.-%, oder bevorzugt zu mindestens 90 Gew.-%, bezogen auf das Gesamtgewicht der Komponente C2.. Besonders bevorzugt besteht die Komponente C2. aus ausschließlich anionisch hydrophilierenden Verbindungen.

Geeignete anionisch hydrophilierende Verbindungen enthalten wenigstens eine anionische oder ionogene Gruppe, die in eine anionische Gruppe überführt werden kann. Des Weiteren bevorzugt weisen geeignete anionisch hydrophilierende Verbindungen wenigstens zwei Aminogruppen und besonders bevorzugt zwei Aminogruppen auf. Besonders bevorzugt umfasst die hydrophilierende Komponente C2. eine anionisch hydrophilierende Verbindung, die wenigstens eine anionische oder ionogene Gruppe und wenigstens zwei Aminogruppen aufweist oder besteht daraus.

Geeignete anionisch hydrophilierende Verbindungen als Komponente C2., im Weiteren auch Hydrophilierungsmittel C2. genannt, enthalten bevorzugt eine Sulfonsäure- oder Sulfonatgruppe, besonders bevorzugt eine Natriumsulfonatgruppe. Geeignete anionisch hydrophilierende Verbindungen als Komponente C2. sind insbesondere die Alkalimetallsalze der Mono- und Diaminosulfonsäuren. Beispiele solcher anionischen Hydrophilierungsmittel sind Salze der 2-(2-Aminoethylamino)ethansulfonsäure, Ethylendiamin-propyl- oder -butylsulfonsäure oder 1,2- oder 1,3-Propylendiamin-β-ethylsulfonsäure oder Mischungen aus mindestens zwei hiervon.

Bevorzugte anionische Hydrophilierungsmittel C2. sind solche, die Sulfonatgruppen als ionische Gruppen und zwei Aminogruppen enthalten, wie die Salze der 2-(2-Aminoethylamino)ethylsulfonsäure und 1,3-Propylendiamin-βethylsulfonsäure. Ganz besonders bevorzugt wird 2-(2-Aminoethylamino)ethylsulfonsäure oder deren Salze als anionisches Hydrophilierungsmittel C2. eingesetzt.

Gegebenenfalls kann die anionische Gruppe in der Komponente C2. auch eine Carboxylat- bzw. Carbonsäuregruppe sein. Die Komponente C2. wird dann bevorzugt aus Diaminocarbonsäuren ausgewählt. Bei dieser alternativen Ausführungsform müssen jedoch die Carbonsäure-basierenden Komponenten C2. in höheren Konzentrationen eingesetzt werden, verglichen mit solchen Komponenten C2., die Sulfonat- oder Sulfonsäuregruppen tragen. Besonders bevorzugt werden daher zur Herstellung des Polyurethanharnstoffs keine hydrophilierenden Verbindungen, die ausschließlich Carboxylatgruppen als anionische Gruppen der Komponente C2. tragen eingesetzt.

Bevorzugt wird zur Herstellung des erfindungsgemäß verwendeten Polyurethanharnstoffs in einem Bereich von $\geq 0{,}1$ und $\leq 10$ Gew.-% der Komponente C2. und besonders bevorzugt in einem Bereich von $\geq 0{,}5$ und $\leq 4$ Gew.% der Komponente C2., jeweils bezogen auf die Gesamtmasse des Polyurethanharnstoffs, eingesetzt.

Zur Hydrophilierung können auch Mischungen aus anionischen Hydrophilierungsmitteln C2. und weiteren Hydrophilierungsmitteln D., welche von C2. verschieden sind, verwendet werden.

Geeignete weitere Hydrophilierungsmittel D. sind beispielsweise nichtionische hydrophilierende Verbindungen D1. und/oder hydroxyfunktionelle anionische oder anionogene Hydrophilierungsmittel D2. Bevorzugt handelt es sich bei der Komponente D. um nichtionisch hydrophilierende Komponenten D1..

Geeignete hydroxyfunktionelle ionische oder ionogene Hydrophilierungsmittel als Komponente D2. sind beispielsweise Hydroxycarbonsäuren wie Mono- und Dihydroxycarbonsäuren, wie 2-Hydroxyessigsäure, 3-Hydroxypropansäure, 12-Hydroxy-9-octadecansäure (Rizinolsäure), Hydroxypivalinsäure, Milchsäure, Dimethylolbuttersäure und/oder Dimethylolpropionsäure oder Gemische von mindestens zwei hieraus. Bevorzugt sind Hydroxypivalinsäure, Milchsäure und/oder Dimethylolpropionsäure, besonders bevorzugt ist Dimethylolpropionsäure. Bevorzugt werden keine hydroxyfunktionellen ionische oder ionogenen Hydrophilierungsmittel D2., insbesondere bevorzugt keine Hydrophilierungsmittel, die Carboxylat und Hydroxygruppen aufweisen, wie beispielsweise Dimethylolpropionsäure eingesetzt. Bevorzugt ist die Menge an hydroxyfunktionellen ionischen oder ionogenen Hydrophilierungsmittel D2. in einem Bereich von 0 bis 1 Gew.-%, oder bevorzugt in einem Bereich von 0 bis 0,5 Gew.-%, bezogen auf die Gesamtmasse des Polyurethanharnstoffs in dem Polyurethanharnstoff enthalten.

Geeignete nichtionisch hydrophilierende Verbindungen als Komponente D1. sind z.B. Polyoxyalkylenether, welche über isocyanatreaktive Gruppen, wie Hydroxy-, Amino- oder Thiolgruppen verfügen. Bevorzugt sind monohydroxyfunktionelle, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind insbesondere gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether, Methanol oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Bevorzugt enthält der verwendete Polyurethanharnstoff zur Bildung der Polyurethanharnstoff-Dispersion (B) in einem Bereich von ≥ 0 und ≤ 20 Gew.-% der Komponente D., bevorzugt in einem Bereich von ≥ 0 und ≤ 10 Gew.-% der Komponente D. und ganz besonders bevorzugt in einem Bereich von ≥ 0 und ≤ 5 Gew.-% der Komponente D., jeweils bezogen auf die Gesamtmasse des Polyurethanharnstoffs. In einer weiteren bevorzugten Ausführungsform wird Komponente D. zur Herstellung des Polyurethanharnstoffs nicht verwendet.

Als Komponente E. können wahlweise Polyole, insbesondere nicht-polymere Polyole, des genannten Molekulargewichtsbereichs von 62 bis 399 mol/g mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Trimethylolethan, Glycerin, Pentaerythrit sowie deren beliebige Mischungen untereinander eingesetzt werden.

Bevorzugt enthält der verwendete Polyurethanharnstoff zur Bildung der Polyurethanharnstoff-Dispersion (B) ≤ 10 Gew.-% der Komponente E., bevorzugt ≤ 5 Gew.-%, besonders bevorzugt 0 Gew.-% der Komponente E., jeweils bezogen auf die Gesamtmasse des Polyurethanharnstoffs. Bevorzugt beinhaltet der Polyurethanharnstoff die Komponente E. in einem Bereich von 0,1 bis 10 Gew.-%, bevorzugt in einem Bereich von 0,2 bis 8 Gew.-%, bevorzugt in einem Bereich von 0,1 bis 5 Gew.-%, bezogen jeweils auf die Gesamtmasse des Polyurethanharnstoffs. In einer weiteren bevorzugten Ausführungsform wird Komponente E. zur Herstellung des Polyurethanharnstoffs nicht verwendet.

Bevorzugt wird zur Herstellung des erfindungsgemäß verwendeten Polyurethanharnstoffs in einem Bereich von ≥ 0,5 und ≤ 20 Gew.-% der Summe der Komponenten C1. und gegebenenfalls E. oder bevorzugt in einem Bereich von ≥ 1 und ≤ 15 Gew.-% der Summe der Komponenten C1. und gegebenenfalls E., jeweils bezogen auf die Gesamtmasse des Polyurethanharnstoffs, eingesetzt.

Als Komponente F. können weitere polymere Polyole, welche unterschiedlich sind von B. eingesetzt werden.

Beispiele sind polymere Polyole, die nicht unter die Definition von B. fallen, weil sie keine Polyetherpolyole sind - beispielsweise die in der Polyurethanlacktechnologie an sich bekannten Polyesterpolyole, Polyacrylatpolyole, Polyurethanpolyole, Polycarbonatpolyole, , Polyesterpolyacrylatpolyole, Polyurethanpolyacrylatpolyole, Polyurethanpolyesterpolyole, , Polyurethanpolycarbonatpolyole und Polyesterpolycarbonatpolyole.

Bevorzugt handelt es sich bei der Komponente F. nicht um polymere Polyole die Estergruppen aufweisen, insbesondere nicht um Polyesterpolyole.

Die Komponenten B. und F. enthalten erfindungsgemäß zusammen ≤ 30 Gew.-%, bevorzugt ≤ 10 Gew.-%, und besonders bevorzugt ≤ 5 Gew.-%, an Komponente F., bezogen auf die Gesamtmasse der Komponenten B. und F.. Ganz besonders bevorzugt wird die Komponente F. zur Herstellung des Polyurethanharnstoffs nicht eingesetzt.

Bevorzugt wird zur Herstellung des erfindungsgemäß verwendeten Polyurethanharnstoffs in einem Bereich von ≥ 55 und ≤ 90 Gew.-% der Summe der Komponenten B. und gegebenenfalls F. und besonders bevorzugt in einem Bereich von ≥ 60 und ≤ 85 Gew.-% der Summe der Komponenten B. und gegebenenfalls F., jeweils bezogen auf die Gesamtmasse des Polyurethanharnstoffs, eingesetzt.

Bei der Komponente G. handelt es sich um Verbindungen, die genau eine isocyanatreaktive Gruppe aufweisen, oder um Verbindungen, die mehr als eine isocyanatreaktive Gruppe aufweisen, wobei nur eine der isocyanat reaktiven Gruppen unter den gewählten Umsetzungbedingungen mit den in der Reaktionsmischung vorhandenen Isocyanatgruppen reagiert.

Bei den isocyanatreaktiven Gruppen der Komponente G. kann es sich dabei um jede funktionelle Gruppe handeln, die mit einer Isocyanatgruppe reagieren kann, wie beispielsweise Hydroxygruppen, Thiolgruppen oder primäre und sekundäre Aminogruppen.

Isocyanatreaktive Gruppen im Sinne der Erfindung sind dabei insbesondere bevorzugt primäre oder sekundäre Aminogruppen, die mit Isocyanatgruppen unter Bildung von Harnstoffgruppen reagieren. Neben der Aminogruppe können die Verbindungen der Komponente G. auch andere prinzipiell isocyanatreaktive Gruppen, wie OH-Gruppen aufweisen, wobei nur eine der isocyanatreaktiven Gruppen unter den gewählten Umsetzungbedingungen mit den in der Reaktionsmischung vorhandenen Isocyanatgruppen reagiert. Dies kann beispielsweise durch Reaktion entsprechender Aminoalkohole bei relativ niedrigen Temperaturen erfolgen, beispielsweise bei 0 bis 60°C, bevorzugt bei 20 bis 40°C. Bevorzugt wird dabei in Abwesenheit von Katalysatoren gearbeitet, welche die Reaktion von Isocyanatgruppen mit Alkoholgruppen katalysieren würden.

Beispiele für geeignete Verbindungen der Komponente G. sind primäre/sekundäre Amine, wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, Diethanolamin, 3-Amino-1-methylaminopropan, 3-Amino-1-ethylaminopropan, 3-Amino-1-cyclohexylaminopropan, 3-Amino-1- methylaminobutan, Ethanolamin, 3-Aminopropanol oder Neopentanolamin.

Geeignete monofunktionellen Verbindungen sind auch Ethanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmonopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Bevorzugt wird zur Herstellung des erfindungsgemäß verwendeten Polyurethanharnstoffs ≥ 0,1 und ≤ 20 Gew.-% der Komponente G. und besonders bevorzugt ≥ 0,3 und ≤ 10 Gew.-% der Komponente G., jeweils bezogen auf die Gesamtmasse des Polyurethanharnstoffs, eingesetzt.

Bevorzugt wird die Komponente H. eingesetzt und das molare Verhältnis von Komponente G. zu Komponente H. beträgt bevorzugt 5:1 bis 1:5, besonders bevorzugt 1,5:1 bis 1:4 und ganz besonders bevorzugt 1:1 bis 1:3.

In einer bevorzugten Ausführungsform des Hygieneartikels weist mindestens die Absorptionsschicht oder die Speicherschicht oder beide, eine Feuchtigkeitsaufnahmerate in einem Bereich von 20 µl/s bis 500 µl/s, oder bevorzugt in einem Bereich von 50 µl/s bis 300 µl/s, oder bevorzugt in einem Bereich von 70 µl/s bis 200 µl/s auf, jeweils ermittelt gemäß der unter Mess-Methoden angegebenen Methode. Bevorzugt weist die Absorptionsschicht eine Feuchtigkeitsaufnahmerate auf, die größer ist als die der Speicherschicht, bevorzugt um das 1,5 bis 7 fache größer ist als die Feuchtigkeitsaufnahmerate der Speicherschicht.

Der Hygieneartikel kann neben der Absorptionsschicht und der Speicherschicht mindestens eine weitere Schicht aufweisen ausgewählt aus der Gruppe bestehend aus einer Deckschicht, einer Hautkontaktschicht, einer wasserabweisenden Schicht, eine schmutzabweisenden Schicht, einem Vliesstoff, einer superabsorbierenden Schicht oder einer Kombination aus mindestens zwei hieraus. Ebenso kann die Speicherschicht bestimmte weitere Materialien wie einen Vliesstoff oder einen Superabsorber enthalten, welche direkt beim Herstellen der Speicherschicht in diese eingearbeitet werden. Diese weiteren Schichten sind in dem Hygieneartikel bevorzugt auf der Seite der Speicherschicht angeordnet. Die mindestens eine weitere Schicht wird bevorzugt unmittelbar auf die Speicherschicht aufgebracht. Bevorzugt ist die Absorptionsschicht so ausgestaltet, dass sie als Hautkontaktschicht dienen kann.

Ein weiterer Gegenstand der Erfindung ist ein Verfahren zur Herstellung eines Hygieneartikels, mit den Schritten

(V1) mechanisches Aufschäumen einer Polyurethandispersion a) zu einem Polyurethanschaum (A) unter Bildung einer Absorptionsschicht und anschließendem mindestens teilweisen Trocknen des Polyurethanschaums (A);

(V2) Erzeugen eines Polyurethanschaums (B) in Form einer Speicherschicht in direktem Kontakt mit mindestens einem Teil der Absorptionsschicht, wobei der Polyurethanschaum (B) durch reaktives Umsetzen wenigstens der folgenden Komponenten hergestellt wird:

A) isocyanatfunktionelle Präpolymere erhältlich durch die Umsetzung von A1)niedermolekularen Diisocyanaten einer Molmasse von 140 bis 278 g/mol, mit A2)Polyalkylenoxiden mit einer OH-Funktionalität von zwei oder mehr, A3) optional weitere isocyanatreaktive Komponenten, die nicht unter A2) fallen;

B) Wasser in einer Menge von mindestens 2 Gew.-%, bezogen auf das Gesamtgewicht der Zusammensetzung;

C) optional heterocyclische, 4-Ring- oder 6-Ring-Oligomere von niedermolekularen Diisocyanaten mit einer Molmasse von 140 bis 278 g/mol,

D) optional Katalysatoren;

E) optional Salze schwacher Säuren deren korrespondierende freie Säuren in Wasser bei 25 °C einen pKS-Wert von ≥ 3,0 und ≤ 14,0 aufweisen;

F) optional Tenside; und

G) optional ein- oder mehrwertige Alkohole oder Polyole;

H) optional hydrophile Polyisocyanate erhältlich durch Umsetzung von

H1)niedermolekularen Diisocyanaten einer Molmasse von 140 bis 278 g/mol und / oder daraus herstellbaren Polyisocyanaten mit einer Isocyanatfunktionalität von 2 bis 6, mit

H2)monofunktionellen Polyalkylenoxiden einer OH-Zahl von 10 bis 250, und einem Ethylenoxidanteil von 50 bis 100 mol%, bezogen auf die Gesamtmenge der enthaltenen Oxyalkylengruppen.

bereitgestellt, aufgeschäumt und ausgehärtet werden.

Die Auswahl und Ausgestaltung wie Mengen, Verhältnisse und andere Eigenschaften der Komponenten A) bis H) zur Herstellung des Polyurethanschaums (B) sind die gleichen wie für den zuvor beschriebenen erfindungsgemäßen Hygieneartikel und daher dem zuvor beschriebenen Komponenten A) bis H) zu entnehmen. Die Auswahl und Ausgestaltung wie Mengen, Verhältnisse und andere Eigenschaften von Komponenten aus denen die Polyurethandispersion a) hergestellt wird, sind die gleichen wie im Zusammenhang mit der Polyurethandispersion a) für den zuvor beschriebenen erfindungsgemäßen Hygieneartikel und daher ebenfalls für die Herstellung der Polyurethandispersion a) gültig.

Das mechanische Aufschäumen in Schritt (V1) kann auf jede Weise erfolgen, die der Fachmann hierfür vorsehen würde. Bevorzugt wird das mechanische Aufschäumen mit Hilfe eines geeigneten Mischaggregats wie z.B. die von der Fa. Hansa-Mixer angebotenen Mischaggregate oder einem Oszillationsmischrohr durchgeführt. Im ersten Fall wird im Mischkopf eine definierte Menge Luft in die Polyurethandispersion a) eingedrückt und mittels Stachelwalze wird die Luft fein verteilt, so dass die gewünschte Schaumdichte direkt einstellbar ist. Im zweiten Fall wird die Dispersion durch ein zyklisches Rohr (in Größe und Form einem Donut ähnelnd) gepumpt, das im Inneren Einbauten/ Fortsätze hat, die zu einer Störung der Strömung führen. Gleichzeitig wird das Rohr geschüttelt oder anderen mechanischen Bewegungen ausgesetzt und Luft in die Dispersion eingeblasen.

Das Erzeugen des Polyurethanschaums (B) in Schritt (V2) kann auf jede Weise erfolgen, die der Fachmann hierfür vorsehen würde. Bevorzugt erfolgt das Erzeugen in Schritt (V2) mittels Rakeln, Gießen, Sprühen, Drucken, Pinseln oder einer Kombination aus mindestens zwei hiervon.

Bei der Herstellung der isocyanatfunktionellen Präpolymere A) wird das Verhältnis der Polyalkylenoxide A2) zu den niedermolekularen, aliphatischen Diisocyanaten A1) typischerweise so eingestellt, dass auf 1 Mol OH-Gruppen des Polyalkylenoxide A2) 1,1 bis 20 Mol, bevorzugt mit 1,3 bis 5 Mol und besonders bevorzugt 1,5 bis 3,5 NCO-Gruppen des niedermolekularen, aliphatischen Diisocyanats A1) kommen.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140 °C, bevorzugt 60 bis 100 °C.

Wurde mit überschüssigem Isocyanat gearbeitet, so erfolgt anschließend die Entfernung des Überschusses an niedermolekularem, aliphatischen Diisocyanat bevorzugt durch Dünnschichtdestillation.

In einer bevorzugten Ausführungsform des Verfahrens wird die Polyurethandispersion a) durch Umsetzung wenigstens der folgenden Komponenten hergestellt:

a1) ein isocyanatfunktionelles Prepolymer,

a2) gegebenenfalls ein isocyanatreaktives, bevorzugt aminofunktionelles, anionisches oder potentiell anionisches Hydrophilierungsmittel,

a3) gegebenenfalls eine aminofunktionelle Verbindung, insbesondere mit einem Molekulargewicht von 32 bis 400 g/mol,

a4) Wasser,

wobei das Hydrophilierungsmittel a2) dann obligatorisch ist, wenn das Prepolymer a1) keine hydrophilierenden Gruppen aufweist.

In einer bevorzugten Ausführungsform des Verfahrens beinhaltet die Polyurethandispersion a) zwei unterschiedliche Polyurethan-Dispersionen (A) und (B). Sämtliche Merkmale, wie der Aufbau, die Art, Menge und Anteile der Komponenten, die Verhältnisse der Dispersionen (A) und (B) entsprechen denen, wie für den Hygieneartikel zuvor beschrieben und sind für das Verfahren gleichfalls gültig.

In einer bevorzugten Ausführungsform des Verfahrens wird der Polyurethanschaum (A) auf den bereits fertigen oder den in Bildung befindlichen Polyurethanschaum (B) oder der Polyurethanschaum (B) auf den bereits fertigen oder den in Bildung befindlichen Polyurethanschaum (A) aufgebracht bzw. mit diesem in Kontakt gebracht.

Bevorzugt wird zunächst der Polyurethanschaum (A) zubereitet und zumindest zu einem Teil getrocknet. Der Polyurethanschaum (B) kann in einer ersten Alternative (AI) auf den getrockneten oder zum Teil getrockneten Polyurethanschaum (A) aufgebracht werden oder in einer zweiten Alternative (AII) bei seiner Herstellung mit dem zumindest teilweise getrockneten Polymerschaum (A) in Kontakt gebracht werden. Der Polyurethanschaum (A) muss dabei noch nicht vollständig getrocknet sein. Der Begriff "noch nicht vollständig getrocknet" oder "in Bildung befindliche" bedeutet gemäß der Erfindung, dass der jeweilige Schaum, wie der Polyurethanschaum (A) oder der Polyurethanschaum (B), der jeweils als erstes gebildet wird, noch einen Restfeuchtegehalt von 20 Gew.-%, oder bevorzugt von höchstens 15 Gew.-%, oder bevorzugt von höchstens 10 Gew.-%, bezogen auf die Gesamtmasse des Polyurethanschaums (A) bzw. (B) aufweist.

In der zweiten Alternative (AII) werden zunächst alle ausgewählten Komponenten A) bis H) zusammengefügt und die erhaltene Mischung (V) auf ein Substrat gegeben. Bevorzugt wird innerhalb von 0,1 bis 20 Sekunden nach Aufgabe der Komponenten A) bis H) zur Bildung des Polyurethanschaums (B) auf das Substrat, der Polyurethanschaum (A) auf die dem Substrat, beispielsweise in Form eines Trennpapiers, gegenüberliegende Seite des Polyurethanschaums (B) gelegt.

In einer bevorzugten Ausführungsform des Verfahrens wird die aufgeschäumte Polyurethandispersion a) auf den Polyurethanschaum (B) aufgebracht und dort getrocknet.

In einer bevorzugten Ausführungsform des Hygieneartikel, der bevorzugt nach dem zuvor beschriebenen Verfahren hergestellt ist, kann der Hygieneartikel zusätzlich eine Deckschicht aufweisen, die insbesondere mit der Speicherschicht verbunden sein kann.

In einer bevorzugten Ausführungsform des Hygieneartikels umfasst die Deckschicht ein thermoplastisches Polyurethan und / oder ein thermoplastisches Polyethylen oder besteht daraus.

**Mess-Methoden**

Absorptionsvermögen

Das maximale Absorptionsvermögen des Hygieneartikels, bzw. der einzelnen Schaumschichten, wie der Absorptionsschicht oder der Speicherschicht wurde gemäß DIN EN 13726-1 jeweils an einem Schaumstück der Größe 5 x 5 cm$^2$ bestimmt. Die aufgenommene Flüssigkeit wird in % des Trockengewichtes des Schaumes der jeweiligen Schicht angegeben, wobei das Trockengewicht des Schaumes 100% entspricht, entsprechend:

$$Fl\ddot{u}ssigkeitsabsorption\ in\ \% = 100 \cdot \frac{Masse\ nach\ Absorption - Masse\ vor\ Absorption (Trockengewicht)}{Masse\ vor\ Absorption}$$

Retention

Für die Bestimmung der Retention wird ein Schaumstück der Größe 5 x 5 cm$^2$ nach vollständiger Absorption mit einer Pinzette angehoben und für 30 Sekunden abtropfen gelassen und gewogen. Danach wird das Schaumstück auf ein Metallpodest gelegt und mit einem Gewicht von 6 kg für 20 Sekunden beschwert. Nach Entfernen des Gewichtes wird das Schaumstück erneut gewogen und die verbliebene Feuchtigkeitsmenge im Vergleich prozentual zur maximalen Absorption bestimmt.

Feuchtigkeitsaufnahmerate (Wicking):

Die Feuchtigkeitsaufnahmerate ist ein Maß für die Geschwindigkeit eines Materials Wasser aufzunehmen. Dabei werden 30 $\mu$l der eingefärbten Prüflösung A* (mit 0,1 Gew.-% Dragocolor (E133)), die zuvor auf 37°C erwärmt wurde, auf die zu untersuchende Schaumoberfläche mittels einer Eppendorf Pipette aufgesetzt. Die Eindringzeit des Tropfens wird mittels einer Stoppuhr ermittelt. Der Start erfolgt sobald der Tropfen aufgesetzt wurde. Die Stoppuhr wird gestoppt, wenn der Tropfen vollständig eingedrungen ist. Der Tropfen gilt als vollständig eingedrungen, wenn die Tropfenoberfläche keine Reflektion im Licht (beispielsweise einer verwendeten Lampe) mehr zeigt. Die Messung erfolgt in 5fach-Bestimmung.

*Prüflösung A: eine NaCl und CaCl$_2$ mit einem Gehalt von 142 mmol Natriumionen und 2,5 mmol Calciumionen als Chloride. Diese Lösung weist eine dem menschlichen Serum oder Wundsekret vergleichbare Ionenzusammensetzung auf Sie wird durch Lösen von 8,298 g Natriumchlorid und 0,368 g Calciumchlorid-Dihydrat in deionisiertem Wasser und Auffüllen auf 1 l in einem Messkolben hergestellt.*

Dickenmessung:

Die Schichtdickenbestimmung wurde mit einem Drucklufttaster und zur Ausgabe der Schichtdicke angeschlossenem Display der Firma Heidehain (MT25P) ermittelt.

Dichtemessung:

Zur Dichtebestimmung wurde ein Probenstück mit Hilfe eines Stanzeisens in der Dimension 5 x 5 cm$^2$ (mit abgerundeten Ecken und Kurvenradius von 3 mm) ausgestanzt. Die Höhe wurde aus dem Mittelwert einer 5fach-Bestimmung mittels oben beschriebener Mess-Methode ermittelt. Zur anschließenden Berechnung der Dichte wurde die Masse des Probenstückes an einer Mettler Toledo XS603S Waage bestimmt.

Bestimmung der Flächenausdehung

Gemäß DIN EN 13726-1:2002 wurden Schaumstücke der Kantenlänge 5x5 cm$^2$ aus dem hergestellten Schaum ausgestanzt. Dann wurde die Absorption bestimmt und direkt im Anschluss wurde die Kantenlänge der gequollenen Schaumstücke erneut bestimmt und der arithmetische Mittelwert aus den vier Werten gebildet. Die Flächenausdehung wurde wie folgt berechnet.

$$\text{Flächenausdehung } [\%] = 100 \cdot ((\text{mittlere Kantenlänge nach Quellung in cm - 5cm})/5\text{cm})$$

Es wurde eine Doppelbestimmung vorgenommen und der arithmetische Mittelwert aus beiden Messwerten gebildet. Der Quellungsgrad oder die Flächenausdehnung wurde anhand der Kanten vorgenommen, die sich in der Ebene befinden und damit nicht der zusätzlichen Kraft der Schwerkraft ausgesetzt sind.

Bestimmung der Quellung / Volumenquellung

Gemäß DIN EN 13726-1:2002 wurden Schaumstücke der Kantenlänge 5x5 cm$^2$ aus dem hergestellten Schaum ausgestanzt und die Höhe an fünf Stellen des Probenkörpers bestimmt, um das Volumen des trockenen Schaums zu berechnen. Dann wurde die Absorption bestimmt und direkt im Anschluss wurde die Kantenlänge der gequollenen Schaumstücke und Schaumhöhe erneut bestimmt und der arithmetische Mittelwert aus den Werten gebildet. Die Quellung in x, y und z-Richtung, auch Volumenquellung genannt, wurde wie folgt berechnet.

$$\text{Quellung } [\%] = 100 \cdot ((\text{mittlere Volumen nach Quellung in cm} - \text{Volumen vor Quellung})/\text{Volumen vor Quellung})$$

Es wurde eine Doppelbestimmung vorgenommen und der arithmetische Mittelwert aus beiden Messwerten gebildet. Der Quellungsgrad oder die Längsausdehnung wurde anhand der Kanten vorgenommen, die sich in der Ebene befinden und damit nicht der zusätzlichen Kraft der Schwerkraft ausgesetzt sind.

**Herstellung der Präpolymere, Polyurethandispersionen**

Zur Herstellung der nachfolgend genannten Präpolymere und Polyurethandispersionen wurden, wenn nicht anders bezeichnet Chemikalien verwendet, die bei Sigma-Aldrich erhalten wurden.

**Beispiel für Präpolymer PPA1)**

Für ein erstes Beispiel PPA1) des Präpolymer A) als Ausgangsstoff zur Bildung des Polyurethanschaums (B) einer Speicherschicht wurde Baymedix® FP505, ein Handelsprodukt der Covestro Deutschland AG, verwendet.

**Beispiel für die Herstellung des Präpolymers PPA2)**

Zu einem Gemisch aus 1680 g HDI und 5.0 g Dibutylphosphat wurde bei 80 °C innerhalb von 30 min 2960 g eines Polyalkylenoxids mit einer Molmasse von 591 g/mol (OH-Zahl 190 mgKOH/g), gestartet auf 1,3-Propylenglycol und einem Ethylenoxidgewichtsanteil von 87% zugetropft und nachgerührt bis ein NCO-Gehalt von 9,1% erreicht wurde (3,5 h). Das überschüssige HDI wurde durch Dünnschichtdestillation bei 140 °C und 0,7 mbar entfernt. Man erhielt ein Präpolymer mit einem NCO-Gehalt von 5,0 % und einer Viskosität von 5140 mPas. Der berechnete Urethangehalt beträgt 2,4 mol/kg.

**Herstellung der wässrigen Polyurethandispersion a), Beispiel Polyurethan-Dispersion (A)**

1077,2 g PolyTHF® 2000, 409,7 g PolyTHF® 1000, 830,9 g Desmophen® C2200 und 48,3 g Polyether LB 25 wurden in einer Standard-Rührapparatur auf 70 °C aufgeheizt. Anschließend wurde bei 70 °C innerhalb von 5 min ein Gemisch aus 258,7 g Hexamethylendiisocyanat und 341,9 g Isophorondiisocyanat zugegeben und bei 120°C gerührt, bis der theoretische NCO-Wert erreicht oder leicht unterschritten war. Das fertige Prepolymer wurde mit 4840 g Aceton gelöst und dabei auf 50 °C abgekühlt und anschließend wurde eine Lösung aus 27,4 g Ethylendiamin, 127,1 g Isophorondiamin, 67,3 g Diaminosulfonat und 1200 g Wasser innerhalb von 10 min zudosiert. Die Nachrührzeit betrug 10 min. Dann wurde durch Zugabe von 654 g Wasser dispergiert. Es folgte die Entfernung des Lösemittels durch Destillation im Vakuum.

Die erhaltene Polyurethan-Dispersion hatte nachfolgende Eigenschaften:

| | |
|---|---|
| Feststoffgehalt: | 61,6 % |
| Partikelgröße (LKS): | 528 nm |
| pH (23°C): | 7,5 |

**Herstellung der wässrigen Polyurethandispersion a), Beispiel Polyurethan-Dispersion (B)**

75 g PolyTHF® 1000 und 350 g PolyTHF® 2000 wurden auf 70°C aufgeheizt. Anschließend wurde ein Gemisch aus 33,9 g Hexamethylendiisocyanat, 49,7 g Isophorondiisocyanat, sowie 8,7 g Desmodur N 3300 (HDI-Trimerisat mit einem NCO-Gehalt von ca. 21,8% nach DIN EN ISO 11 909) zugegeben und solange bei 100-115°C gerührt bis der theoretische NCO-Wert unterschritten war. Das fertige Präpolymer wurde mit 920 g Aceton bei 50°C gelöst und anschließend eine Lösung aus 3,2 g Ethylendiamin, 12,9 g Diaminosulfonat, 11,7 g Diethanolamin und 145 g Wasser zudosiert. Die Nachrührzeit betrug 15 min. Danach wurde durch Zugabe von 1080 g Wasser dispergiert. Es folgte die Entfernung des Lösemittels durch Destillation im Vakuum und es wurde eine lagerstabile Dispersion erhalten; der Festgehalt wurde durch Zusatz von Wasser eingestellt.

| | |
|---|---|
| Feststoffgehalt: | 52 % |
| Partikelgröße (LKS): | 307 nm |
| Viskosität: | 105 mPa s |
| Tg Polyurethanharnstoff: | -78,0 °C |

**Herstellung Hygieneartikel: Polyurethanschaum (A) auf Polyurethanschaum (B)**

Herstellung des Polyurethanschaums (A)1 als Absorptionsschicht:

3,19 kg der oben beschriebenen wässrigen Polyurethan-Dispersion (A) wurden mit 1,367 kg Polyurethan-Dispersion (B), 0,91 kg einer 10 Gew.-% wässrigen Rheolate 208-Dispersion (der Firma Elementis Specialties, Deutschland) und 0,353 kg einer 40 Gew.-% wässrigen Pluronic PE6800 (der Firma BASF SE, Deutschland) (inkl. 3,45 Gew.-% Zitronensäure (der Firma Bernd Kraft, Deutschland))-Lösung mittels eines Dispermaten der Firma VMA-Getzmann GmbH vermischt. Die Mischung wurde anschließend in einem Pico-Mix der Firma Hansa-Mixer zu einem Schaum (Polyurethanschaum (A)) mit einer Dichte von 200 g/l aufgeschlagen. Der aufgeschlagene Schaum wurde auf ein 30 cm breites polyolefin-beschichtetes Trennpaper (Felix Schoeller Y05200) bei einer Rakeleinstellung von 3000 μm aufgegossen. Anschließend wurde bei 130-140 °C für 7 Minuten getrocknet. Der getrocknete Schaum hatte eine Dicke von 2 mm.

Herstellung des Polyurethanschaums (A)2 - Absorptionsschicht:

4,5 kg der oben beschriebenen wässrigen Polyurethan-Dispersion (A) wurden mit 0,225 kg Niax silicone L6889 (der Firma Momentive Performance Materials GmbH, Deutschland) und 0,349 kg einer 40 Gew.-% wässrigen Pluronic PE6800 (der Firma BASF SE, Deutschland) (inkl. 3,45 Gew.-% Zitronensäure (der Firma Bernd Kraft, Deutschland))-Lösung mittels eines Dispermaten der Firma VMA-Getzmann GmbH vermischt. Die Mischung wurde anschließend in einem Pico-Mix der Firma Hansa-Mixer zu einem Schaum (Polyurethanschaum (A)) mit einer Dichte von 200 g/l aufgeschlagen. Der aufgeschlagene Schaum wurde auf ein 30 cm breites polyolefin-beschichtetes Trennpaper (Felix Schoeller Y05200) bei einer Rakeleinstellung von 3000 μm aufgegossen. Anschließend wurde bei 130-140 °C für 7 Minuten getrocknet. Der getrocknete Schaum hatte eine Dicke von 2 mm.

Herstellung des Polyurethanschaums (B)1 - Speicherschicht:

125 g des Präpolymers A), Beispiel PPA1) wurden mit 25 g einer wässrigen Lösung aus 1,3 Gew.-% Natriumhydrogencarbonat (der Firma Fluka, Deutschland), 4,8 Gew.-% Pluronic® PE6800 (der BASF SE, Deutschland), 0,4 Gew.-% Zitronensäure (der Firma Bernd Kraft, Deutschland) unter starkem Rühren für 7s mit Hilfe eines Rührwerks mit Ankerrührblatt (Labordissolver 5 green 037 der Firma Pendraulik GmbH) bei 930 U/min. vermischt und danach auf ein polyolefin-beschichtetes Trennpapier (Felix Schoeller Y05200) mittels eines Rakels von 1,5 mm Spalt in x-y Richtung aufgebracht. Danach wurde die Oberfläche der Reaktionsmischung, die sich in x-y Richtung erstreckt, mit einer genadelten Variante (umfassend Löcher mit einem Durchmesser von 0,1 mm$^2$ und einer Lochdichte von 10 pro cm$^2$) desselben Papiers abgedeckt.

Folgende Messergebnisse wurden für den Polyurethanschaum (B)1 ermittelt:

| Messmethode: | Einheit | Ergebnis: |
|---|---|---|
| Dicke | mm | 5,5 |
| Absorption | % | 1950 |
| Retention | % | 47 |

Herstellung des Polyurethanschaums (B)2 - Speicherschicht:

126,89 g des Präpolymers A), Beispiel PPA2) wurden mit 23,11 g einer wässrigen Lösung aus 1,8 Gew.-% Natriumhydrogencarbonat (der Firma Fluka, Deutschland), 6,4 Gew.-% Pluronic® PE6800 (der BASF SE, Deutschland) unter starkem Rühren für 7s mit Hilfe eines Rührwerks mit Ankerrührblatt (Labordissolver 5 green 037 der Firma Pendraulik GmbH) bei 930 U/min. vermischt und danach auf ein polyolefin-beschichtetes Trennpapier (Felix Schoeller Y05200) mittels eines Rakels von 1,5 mm Spalt in x-y Richtung aufgebracht. Danach wurde die Oberfläche der Reaktionsmischung, die sich in x-y Richtung erstreckt, mit einer genadelten Variante (umfassend Löcher mit einem Durchmesser von 0,1 mm$^2$ und einer Lochdichte von 10 pro cm$^2$) desselben Papiers abgedeckt.

Folgende Messergebnisse wurden für den Polyurethanschaum (B)2 ermittelt:

| Messmethode: | Einheit | Ergebnis: |
|---|---|---|
| Dicke | mm | 5,8 |
| Absorption | % | 1661 |
| Retention | % | 49 |

Beispiel HA1: Herstellung des Polyurethanschaums (B)1 - Speicherschicht (inkl. Verbinden zur Absorptionsschicht mit Polyurethanschaum (A)1):

125 g des Präpolymers A) Beispiel PPA1) wurden mit 25 g einer wässrigen Lösung aus 1,3 Gew.-% Natriumhydrogencarbonat (der Firma Fluka, Deutschland), 4,8 Gew.-% Pluronic® PE6800 (der BASF SE, Deutschland), 0,4 Gew.-% Zitronensäure (der Firma Bernd Kraft, Deutschland) unter starkem Rühren für 7s mit Hilfe eines Rührwerks mit Ankerrührblatt (Labordissolver 5 green 037 der Firma Pendraulik GmbH) bei 930 U/min vermischt und danach auf ein polyolefin-beschichtetes Trennpapier (Felix Schoeller Y05200) mittels eines Rakels von 1,5 mm Spalt in x-y Richtung aufgebracht, unter allmählicher Bildung des Polyurethanschaums (B)1. Kurz nachdem oder kurz bevor der Polyurethanschaum (B)1 sich vollständig ausgebildet hatte, also visuell keine Schaumbildung mehr erkennbar war, wurde die Oberfläche der Reaktionsmischung, die sich in x-y Richtung erstreckt und nicht mit dem Trennpapier verbunden war, sofort mit der Seite der Absorptionsschicht, die mit dem Trennpapier verbunden war, aus Polyurethanschaum (A)1 abgedeckt, um eine dauerhafte Verbindung des Polyurethanschaums (B)1 als Speicherschicht und des Polyurethanschaums (A)1 der Absorptionsschicht zu gewährleisten. Die Messergebnisse zu dem so gebildeten erfindungsgemäßen Hygieneartikels sind in folgender Tabelle zusammen gefasst:

| Messmethode: | Einheit | Ergebnis: |
|---|---|---|
| Dicke | mm | 7,1 |
| Dichte | g/l | 146 |

(fortgesetzt)

| Messmethode: | Einheit | Ergebnis: |
|---|---|---|
| Absorption | % | 1117 |
| Retention | % | 40 |
| Flächenausdehnung | % | 36 |
| Quellung | % | 126 |
| Wicking (gemessen an der Absorptionsschicht im fertigen Hygieneartikel) | (s) | 0,8 |

Beispiel HA2: Herstellung des Polyurethanschaums (B)1 - Speicherschicht (inkl. Verbinden zur Absorptionsschicht mit Polyurethanschaum (A)2):

125 g des Präpolymers A) Beispiel PPA1) wurden mit 25 g einer wässrigen Lösung aus 1,3 Gew.-% Natriumhydrogencarbonat (der Firma Fluka, Deutschland), 4,8 Gew.-% Pluronic® PE6800 (der BASF SE, Deutschland), 0,4 Gew.-% Zitronensäure (der Firma Bernd Kraft, Deutschland) unter starkem Rühren für 7s mit Hilfe eines Rührwerks mit Ankerrührblatt (Labordissolver 5 green 037 der Firma Pendraulik GmbH) bei 930 U/min. vermischt und danach auf ein polyolefin-beschichtetes Trennpapier (Felix Schoeller Y05200) mittels eines Rakels von 1,5 mm Spalt in x-y Richtung aufgebracht,unter allmählicher Bildung des Polyurethanschaums (B)1. Kurz nachdem oder kurz bevor der Polyurethanschaum (B)1 sich vollständig ausgebildet hatte, also visuell keine Schaumbildung mehr erkennbar war, wurde die Oberfläche der Reaktionsmischung, die sich in x-y Richtung erstreckt und nicht mit dem Trennpapier verbunden war, sofort mit der Seite der Absorptionsschicht, die mit dem Trennpapier verbunden war, aus Polyurethanschaum (A)2 abgedeckt, um eine dauerhafte Verbindung des Polyurethanschaums (B)1 als Speicherschicht und des Polyurethanschaums (A)2 der Absorptionsschicht zu gewährleisten. Die Messergebnisse zu dem so gebildeten erfindungsgemäßen Hygieneartikels sind in folgender Tabelle zusammen gefasst:

| Messmethode: | Einheit | Ergebnis: |
|---|---|---|
| Dicke | mm | 6,2 |
| Dichte | g/l | 162 |
| Absorption | % | 911 |
| Retention | % | 54 |
| Flächenausdehnung | % | 30 |
| Quellung | % | 125 |
| Wicking (gemessen an der Absorptionsschicht im fertigen Hygieneartikel) | (s) | 1,2 |

Beispiel HA3: Herstellung des Polyurethanschaums (B)2 - Speicherschicht (inkl. Verbinden zur Absorptionsschicht (A)1):

126,89 g des Präpolymers A) Beispiel PPA2 wurden mit 23,11 g einer wässrigen Lösung aus 1,8 Gew.-% Natriumhydrogencarbonat (der Firma Fluka, Deutschland), 6,4 Gew.-% Pluronic® PE6800 (der BASF SE, Deutschland) unter starkem Rühren für 7s mit Hilfe eines Rührwerks mit Ankerrührblatt (Labordissolver 5 green 037 der Firma Pendraulik GmbH) bei 930 U/min. vermischt und danach auf ein polyolefin-beschichtetes Trennpapier (Felix Schoeller Y05200) mittels eines Rakels von 1,5 mm Spalt in x-y Richtung aufgebracht unter allmählicher Bildung des Polyurethanschaums (B)2. Kurz nachdem oder kurz bevor der Polyurethanschaum (B)1 sich vollständig ausgebildet hatte, also visuell keine Schaumbildung mehr erkennbar war, wurde die Oberfläche der Reaktionsmischung, die sich in x-y Richtung erstreckt und nicht mit dem Trennpapier verbunden war, sofort mit der Seite der Absorptionsschicht, die mit dem Trennpapier verbunden war, aus Polyurethanschaum (A)1 abgedeckt, um eine dauerhafte Verbindung des Polyurethanschaums (B)2 als Speicherschicht und des Polyurethanschaums (A)1 der Absorptionsschicht zu gewährleisten. Die Messergebnisse zu dem so gebildeten erfindungsgemäßen Hygieneartikels sind in folgender Tabelle zusammen gefasst:.

| Messmethode: | Einheit | Ergebnis: |
|---|---|---|
| Dicke | mm | 7,2 |

(fortgesetzt)

| Messmethode: | Einheit | Ergebnis: |
|---|---|---|
| Dichte | g/l | 118 |
| Absorption | % | 1084 |
| Retention | % | 39 |
| Flächenausdehnung | % | 23 |
| Quellung | % | 93 |
| Wicking (gemessen an der Absorptionsschicht im fertigen Hygieneartikel) | (s) | 0,9 |

Beispiel HA4: Herstellung des Polyurethanschaums (B)2 - Speicherschicht (inkl. Verbinden zur Absorptionsschicht (A)2):

126,89 g des Präpolymers A) Beispiel PPA2 wurden mit 23,11 g einer wässrigen Lösung aus 1,8 Gew.-% Natriumhydrogencarbonat (der Firma Fluka, Deutschland), 6,4 Gew.-% Pluronic® PE6800 (der BASF SE, Deutschland) unter starkem Rühren für 7s mit Hilfe eines Rührwerks mit Ankerrührblatt (Labordissolver 5 green 037 der Firma Pendraulik GmbH) bei 930 U/min. vermischt und danach auf ein polyolefin-beschichtetes Trennpapier (Felix Schoeller Y05200) mittels eines Rakels von 1,5 mm Spalt in x-y Richtung aufgebracht, unter allmählicher Bildung des Polyurethanschaums (B)2. Kurz nachdem oder kurz bevor der Polyurethanschaum (B)2 sich vollständig ausgebildet hatte, also visuell keine Schaumbildung mehr erkennbar war, wurde die Oberfläche der Reaktionsmischung, die sich in x-y Richtung erstreckt und nicht mit dem Trennpapier verbunden war, sofort mit der Seite der Absorptionsschicht, die mit dem Trennpapier verbunden war, aus Polyurethanschaum (A)2 abgedeckt, um eine dauerhafte Verbindung des Polyurethanschaums (B)2 als Speicherschicht und des Polyurethanschaums (A)2 der Absorptionsschicht zu gewährleisten. Die Messergebnisse zu dem so gebildeten erfindungsgemäßen Hygieneartikels sind in folgender Tabelle zusammen gefasst:

| Messmethode: | Einheit | Ergebnis: |
|---|---|---|
| Dicke | mm | 7,6 |
| Dichte | g/l | 113 |
| Absorption | % | 1081 |
| Retention | % | 40 |
| Flächenausdehnung | % | 20 |
| Quellung | % | 92 |
| Wicking (gemessen an der Absorptionsschicht im fertigen Hygieneartikel) | (s) | 1,9 |

**Patentansprüche**

1. Ein Hygieneartikel umfassend eine Absorptionsschicht und eine zumindest bereichsweise mit der Absorptionsschicht unmittelbar verbundene Speicherschicht, **dadurch gekennzeichnet, dass** die Absorptionsschicht einen Polyurethanschaum (A) umfasst, der durch mechanisches Aufschäumen einer wässrigen Polyurethandispersion a) und anschließendes Trocknen erhältlich ist und die Speicherschicht einen Polyurethanschaum (B) umfasst, wobei der Polyurethanschaum (B) durch reaktive Umsetzung wenigstens folgender Komponenten erhältlich ist:

   A) Präpolymere erhältlich durch die Umsetzung von

      A1) niedermolekularen Diisocyanaten einer Molmasse von 140 bis 278 g/mol, mit
      A2) Polyalkylenoxiden mit einer OH-Funktionalität von zwei oder mehr,
      A3) optional weitere isocyanatreaktive Komponenten, die nicht unter A2) fallen;

   B) Wasser in einer Menge von mindestens 2 Gew.-%, bezogen auf das Gesamtgewicht der Zusammensetzung;
   C) optional heterocyclische, 4-Ring- oder 6-Ring-Oligomere von niedermolekularen Diisocyanaten mit einer Molmasse von 140 bis 278 g/mol,

D) optional Katalysatoren;

E) optional Salze schwacher Säuren deren korrespondierende freie Säuren in Wasser bei 25 °C einen pKS-Wert von ≥ 3,0 und ≤ 14,0 aufweisen;

F) optional Tenside; und

G) optional ein- oder mehrwertige Alkohole oder Polyole;

H) optional hydrophile Polyisocyanate erhältlich durch Umsetzung von H

1)niedermolekularen Diisocyanaten einer Molmasse von 140 bis 278 g/mol und / oder daraus herstellbaren Polyisocyanaten mit einer Isocyanatfunktionalität von 2 bis 6, mit

H2)monofunktionellen Polyalkylenoxiden einer OH-Zahl von 10 bis 250, und einem Ethylenoxidanteil von 50 bis 100 mol%, bezogen auf die Gesamtmenge der enthaltenen Oxyalkylengruppen.

2. Der Hygieneartikel nach Anspruch 1, wobei die isocyanathaltigen Komponenten ausgewählt aus den Komponente A) bis H), insbesondere die Komponenten A), C) und H), insgesamt einen Isocyanatgehalt in einem Bereich von 2 bis 8 Gew.-% und einen Gehalt an Urethangruppen von 1,0 bis 3,5 mol/kg, jeweils bezogen auf die Gesamtmenge der isocyanathaltigen Komponenten, aufweisen.

3. Der Hygieneartikel nach einem der vorherigen Ansprüche, wobei die Speicherschicht, die Absorptionsschicht oder der Hygieneartikel mindestens eine der folgenden Eigenschaften aufweist:

a. das Verhältnis des Volumens der Absorptionsschicht zu dem Volumen der Speicherschicht in einem Bereich von 1:1 bis 1:50 liegt, bevorzugt in einem Bereich von 1:1,5 bis 1:20, oder bevorzugt in einem Bereich von 1:2 bis 1:10;

b. die Speicherschicht eine Dicke in einem Bereich von 0,1 bis 20 mm, oder bevorzugt in einem Bereich von 0,35 bis 10 mm, oder bevorzugt in einem Bereich von 0,5 bis 5 mm aufweist;

c. die Speicherschicht ein maximales Absorptionsvermögen für Wasser von ≥ 1000%, oder bevorzugt von ≥ 1200%, oder bevorzugt von ≥ 1500% aufweist (jeweils g/g nach DIN EN 13726-1:2002);

d. die Speicherschicht eine Retention von ≥ 30%, bevorzugt ≥ 40% aufweist , ermittelt nach maximaler Absorption gemäß DIN EN 13726-1:2002 durch Belastung mit 6 kg Gewicht über 20 s auf einem Schaumstück dessen Maße in trockenem Zustand 5 x 5 cm$^2$ betragen;

e. die Absorptionsschicht eine Dicke in einem Bereich von 0,05 bis 5 mm aufweist, oder bevorzugt in einem Bereich 0,2 bis 3 mm, oder bevorzugt in einem Bereich von 0,5 bis 2,5 mm;

f. die Absorptionsschicht eine Feuchtigkeitsaufnahmerate (Wicking) für einen Tropfen Testflüssigkeit von 30 µl von < 2 s, bevorzugt < 1,5 s, oder bevorzugt < 1 s aufweist;

g. der Hygieneartikel eine Erhöhung der Flächenausdehnung bei Kontakt mit Wasser in einem Bereich von ≥ 0,1 % bis ≤ 40 %, oder bevorzugt in einem Bereich von ≥ 0,5 % bis ≤ 38 %, oder bevorzugt in einem Bereich von ≥ 1 % bis ≤ 36 %im Vergleich zur ursprünglichen Flächenausdehnung aufweist;

h. die Speicher- und die Absorptionsschicht untrennbar miteinander verbunden sind;

i. die Speicherschicht, die Absorptionsschicht oder der Hygieneartikel bei einer Absorptionsprüfung nach DIN EN 13726-1:2002 eine Volumenquellung von ≤ 170 %, bevorzugt von ≤ 150 %, oder bevorzugt von ≤ 130 %, oder aufweist.

4. Der Hygieneartikel nach Anspruch 1, 2 oder 3, wobei die Polyurethandispersion a) zwei unterschiedliche Polyurethan-Dispersionen (A) und (B) beinhaltet.

5. Der Hygieneartikel nach Anspruch 4, wobei die Polyurethan-Dispersion (A) erhältlich ist, indem

A) isocyanatfunktionelle Prepolymere aus

A1) organischen Polyisocyanaten

A2) polymeren Polyolen mit zahlenmittleren Molekulargewichten von 400 bis 8000 g/mol und OH-Funktionalitäten von 1,5 bis 6 und

A3) gegebenenfalls hydroxyfunktionellen Verbindungen mit Molekulargewichten von 62 bis 399 g/mol und

A4) gegebenenfalls isocyanatreaktiven, anionischen oder potentiell anionischen und gegebenenfalls nichtionischen Hydrophilierungsmitteln,

hergestellt, und

B) deren freie NCO-Gruppen dann ganz oder teilweise

B1) gegebenenfalls mit aminofunktionellen Verbindungen mit Molekulargewichten von 32 bis 400 g/mol und
B2) mit aminofunktionellen, anionischen oder potentiell anionischen Hydrophilierungsmitteln

unter Kettenverlängerung umgesetzt werden und die Prepolymere vor, während oder nach Schritt B) in Wasser dispergiert werden.

6. Der Hygieneartikel nach Anspruch 4 oder 5, wobei die Polyurethan-Dispersion (B) erhältlich ist, durch reaktive Umsetzung wenigstens folgender Komponenten:

A. einer aliphatischen Polyisocyanat-Komponente mit einer mittleren Isocyanatfunktionalität von $\geq 1,8$ und $\leq 2,6$,
B. einer polymeren Polyether-Polyol-Komponente;
C. einer aminofunktionellen Kettenverlängerer-Komponente mit mindestens 2 isocyanatreaktiven Aminogruppen, enthaltend wenigstens eine aminofunktionelle Verbindung C1., die keine ionischen oder ionogenen Gruppen aufweist und/oder eine aminofunktionelle Verbindung C2., die anionische oder anionogene Gruppen aufweist,
D. gegebenenfalls weiteren hydrophilierenden Komponenten, die unterschiedlich sind von C2.,
E. gegebenenfalls hydroxyfunktionellen Verbindungen mit einem Molekulargewicht von 62 bis 399 mol/g,
F. gegebenenfalls weiteren polymeren Polyolen, welche unterschiedlich sind von B.,
G. einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und
H. optional einer aliphatischen Polyisocyanat-Komponente mit einer mittleren Isocyanatfunktionalität von > 2,6 und $\leq 4$,

wobei die Komponenten B. und F. zusammen $\leq 30$ Gew.-% an Komponente F., bevorzugt $\leq 20$ Gew.-% an Komponente F., oder bevorzugt $\leq 10$ Gew.-% an Komponente F., bezogen auf die Gesamtmasse der Komponenten B. und F. enthalten

7. Der Hygieneartikel nach einem der Ansprüche 1 bis 6, wobei mindestens die Absorptionsschicht oder die Speicherschicht jeweils eine Feuchtigkeitsaufnahmerate in einem Bereich von 20 $\mu$l/s bis 500 $\mu$l/s, oder bevorzugt in einem Bereich von 50 $\mu$l/s bis 300 $\mu$l/s, oder bevorzugt in einem Bereich von 70 $\mu$l/s bis 200 $\mu$l/s aufweist.

8. Ein Verfahren zur Herstellung eines Hygieneartikels mit den Schritten

(V1) mechanisches Aufschäumen einer Polyurethandispersion a) zu einem Polyurethanschaum (A) unter Bildung einer Absorptionsschicht, und anschließendem mindestens teilweisen Trocknen des Polyurethanschaums (A);
(V2) Erzeugen eines Polyurethanschaums (B) in Form einer Speicherschicht auf mindestens einem Teil der Absorptionsschicht, wobei der Polyurethanschaum (B) durch reaktives Umsetzen wenigstens der folgenden Komponenten hergestellt wird:

A) isocyanatfunktionelle Präpolymere erhältlich durch die Umsetzung von

A1) niedermolekularen Diisocyanaten einer Molmasse von 140 bis 278 g/mol, mit
A2) Polyalkylenoxiden mit einer OH-Funktionalität von zwei oder mehr,
A3) optional weitere isocyanatreaktive Komponenten, die nicht unter A2) fallen;

B) Wasser in einer Menge von mindestens 2 Gew.-%, bezogen auf das Gesamtgewicht der Zusammensetzung;
C) optional heterocyclische, 4-Ring- oder 6-Ring-Oligomere von niedermolekularen Diisocyanaten mit einer Molmasse von 140 bis 278 g/mol,
D) optional Katalysatoren;
E) optional Salze schwacher Säuren deren korrespondierende freie Säuren in Wasser bei 25 °C einen pKS-Wert von $\geq 3,0$ und $\leq 14,0$ aufweisen;
F) optional Tenside; und
G) optional ein- oder mehrwertige Alkohole oder Polyole;
H) optional hydrophile Polyisocyanate erhältlich durch Umsetzung von H

1)niedermolekularen Diisocyanaten einer Molmasse von 140 bis 278 g/mol und / oder daraus herstellbaren Polyisocyanaten mit einer Isocyanatfunktionalität von 2 bis 6, mit

H2)monofunktionellen Polyalkylenoxiden einer OH-Zahl von 10 bis 250, und einem Ethylenoxidanteil von 50 bis 100 mol%, bezogen auf die Gesamtmenge der enthaltenen Oxyalkylengruppen;

wobei die Absorptionsschicht und die Speicherschicht zumindest bereichsweise unmittelbar verbunden werden.

9. Das Verfahren nach Anspruch 8, wobei die Polyurethandispersion a) zwei unterschiedliche Polyurethan-Dispersionen (A) und (B) beinhaltet.

10. Das Verfahren nach einem der Ansprüche 8 oder 9, wobei die Komponente
A2) ein di- bis hexafunktionelles, bevorzugt ein tri- bis hexafunktionelles Polyalkylenoxid mit einer OH-Zahl von 22,5 bis 112 und einem Ethylenoxidanteil von 50 bis 100 mol%, bezogen auf die Gesamtmenge der enthaltenen Oxyalkylengruppen beinhaltet.

11. Das Verfahren nach einem der Ansprüche 8 bis 10, wobei der Polyurethanschaum (A) auf den fertigen oder in Bildung befindlichen Polyurethanschaum (B) oder der Polyurethanschaum (B) auf den fertigen oder in Bildung befindlichen Polyurethanschaum (A) aufgebracht wird.

12. Das Verfahren nach einem der Ansprüche 8 bis 11, wobei die aufgeschäumte Polyurethandispersion a) auf den Polyurethanschaum (B) aufgebracht und dort getrocknet wird.

13. Der Hygieneartikel nach einem der Ansprüche 1 bis 7, oder hergestellt nach einem Verfahren gemäß einem der Ansprüche 8 bis 12, wobei der Hygieneartikel zusätzlich eine Deckschicht aufweist, die insbesondere mit der Speicherschicht verbunden sein kann.

14. Der Hygieneartikel nach Anspruch 13, wobei die Deckschicht ein thermoplastisches Polyurethan und / oder ein thermoplastisches Polyethylen umfasst oder daraus besteht.

15. Der Hygieneartikel nach einem der Ansprüche 13 oder 14, oder hergestellt nach einem Verfahren gemäß einem der Ansprüche 8 bis 12, wobei es sich bei dem Hygieneartikel um eine Damenbinde, eine Windel oder einen Inkontinenzartikel handelt.

Europäisches
Patentamt

European
Patent Office

Office européen
des brevets

**EUROPÄISCHER RECHERCHENBERICHT**

**Nummer der Anmeldung**

EP 19 15 9947

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2013/000910 A1 (BAYER IP GMBH [DE]; NIESTEN MEIKE [DE] ET AL.) 3. Januar 2013 (2013-01-03) * Seite 26 - Seite 31; Ansprüche 1-15 * ----- | 1-15 | INV. C08G18/08 A61F13/472 A61F13/537 A61F13/539 |
| A | US 2011/275728 A1 (SCHOENBERGER JAN [DE] ET AL) 10. November 2011 (2011-11-10) * das ganze Dokument * ----- | 1-15 | A61L15/22 C08G18/28 C08G18/40 C08G18/44 C08G18/48 C08G18/72 C08G18/73 C08G18/75 C08G18/79 A61F13/00 A61F13/515 C08G101/00 A61F13/15 |

**RECHERCHIERTE SACHGEBIETE (IPC)**

C08G
A61L
A61F

Der vorliegende Recherchenbericht wurde für alle Patentansprüche erstellt

| Recherchenort | Abschlußdatum der Recherche | Prüfer |
|---|---|---|
| München | 30. August 2019 | Sütterlin, Martin |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes
Dokument

EPO FORM 1503 03.82 (P04C03)

Seite 1 von 2

Europäisches
Patentamt

European
Patent Office

Office européen
des brevets

# EUROPÄISCHER RECHERCHENBERICHT

Nummer der Anmeldung

EP 19 15 9947

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| | | | A61F13/511<br>A61F13/514 |

RECHERCHIERTE
SACHGEBIETE (IPC)

Der vorliegende Recherchenbericht wurde für alle Patentansprüche erstellt

| Recherchenort | Abschlußdatum der Recherche | Prüfer |
|---|---|---|
| München | 30. August 2019 | Sütterlin, Martin |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

................................................................................................

& : Mitglied der gleichen Patentfamilie, übereinstimmendes
    Dokument

EPO FORM 1503 03.82 (P04C03)

Seite 2 von 2

## ANHANG ZUM EUROPÄISCHEN RECHERCHENBERICHT
## ÜBER DIE EUROPÄISCHE PATENTANMELDUNG NR.

EP 19 15 9947

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-08-2019

| Im Recherchenbericht angeführtes Patentdokument | Datum der Veröffentlichung | Mitglied(er) der Patentfamilie | Datum der Veröffentlichung |
|---|---|---|---|
| WO 2013000910 A1 | 03-01-2013 | KEINE | |
| US 2011275728 A1 | 10-11-2011 | AR 074999 A1 | 02-03-2011 |
| | | AU 2010206312 A1 | 18-08-2011 |
| | | BR PI1007319 A2 | 10-02-2016 |
| | | CA 2750424 A1 | 29-07-2010 |
| | | CN 102292365 A | 21-12-2011 |
| | | EP 2389400 A1 | 30-11-2011 |
| | | JP 2012515811 A | 12-07-2012 |
| | | KR 20110117107 A | 26-10-2011 |
| | | TW 201041924 A | 01-12-2010 |
| | | US 2011275728 A1 | 10-11-2011 |
| | | WO 2010083953 A1 | 29-07-2010 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2143744 A1
- EP 2028223 A1
- EP 2140888 A1
- DE 2446440 A
- EP 0916647 A
- WO 0188006 A

**In der Beschreibung aufgeführte Nicht-Patentliteratur**

- Ullmanns Encyclopädie der technischen Chemie. Verlag Chemie, vol. 19, 31-38
- Gelpermeationschromatographie, Teil 1 - Tetrahydrofuran als Elutionsmittel. *SECurity GPC-System von PSS Polymer Service*